(19) Europäisches Patentamt
European Patent Office
Office européen des brevets

(11) Veröffentlichungsnummer: **0 196 005 B1**

(12) # EUROPÄISCHE PATENTSCHRIFT

(45) Veröffentlichungstag der Patentschrift: **20.12.89**

(21) Anmeldenummer: **86103687.9**

(22) Anmeldetag: **18.03.86**

(51) Int. Cl.⁴: **C07D 401/14**, C07D 403/14, C07D 413/14, C07D 417/14, C07D 403/04, C07D 413/04, C07D 417/04, A61K 31/50

(54) **Pyridazinone, ihre Herstellung und diese Verbindungen enthaltende Arzneimittel.**

(30) Priorität: **27.03.85 DE 3511110**

(43) Veröffentlichungstag der Anmeldung: **01.10.86 Patentblatt 86/40**

(45) Bekanntmachung des Hinweises auf die Patenterteilung: **20.12.89 Patentblatt 89/51**

(84) Benannte Vertragsstaaten: **AT BE CH DE FR GB IT LI LU NL SE**

(56) Entgegenhaltungen:
**EP-A- 0 193 013**
**DE-A- 3 129 447**
**US-A- 4 361 563**

**CHEMICAL ABSTRACTS, Band 101, Nr.1, 2. Juli 1984, Columbus, Ohio, USA HONERJAEGER, P.: HEISS, A.; SCHAEFER-KORTING, M. ; SCHOENSTEINER, G.; REITER, M. "UD-CG 115 - a cardiotonic pyridazinone which elevates cyclic AMP and prolongs the action potential in guinea pig papillary muscle" Seite 34, Spalte 2, Zusammenfassung-Nr. 400j 000**
**Medicinal Chemistry, 3 Auflage, Band I, Seite 77**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss)(DE)**

(72) Erfinder: **Hauel, Norbert, Dr. Dipl.-Chem., Händelstrasse 12, D-7950 Biberach 1(DE)**
Erfinder: **Narr, Berthold, Dr. Dipl.-Chem., Obere Au 5, D-7950 Biberach 1(DE)**
Erfinder: **Noll, Klaus, Dr. Dipl.-Chem., Im Schönblick 3, D-7951 Warthausen(DE)**
Erfinder: **Bomhard, Andreas, Dr. Dipl.-Chem., Dinglingerstrasse 9, D-7950 Biberach 1(DE)**
Erfinder: **Heider, Joachim, Dr. Dipl.-Chem., Am Hang 3, D-7951 Warthausen 1(DE)**
Erfinder: **Psiorz, Manfred, Dr. Dipl.-Chem., Riedlinger Strasse 35, D-7950 Biberach 1(DE)**
Erfinder: **Diederen, Willi, Dr., Haldenstrasse 1a, D-7950 Biberach 1(DE)**
Erfinder: **van Meel, Jacques, Dr., Amriswilstrasse 7, D-7950 Biberach 1(DE)**

**EP 0 196 005 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

In der US-Patentschrift 4 026 891, in den Europäischen Patentschriften 0 008 391 und 0 034 743 sowie in der nicht vorveröffentlichten EP-A 0 193 013 werden bereits Pyridazinone beschrieben, welche wertvolle pharmakologische Eigenschaften aufweisen, insbesondere cardiotonische, eine Wirkung auf den Blutdruck und/oder antithrombotische Wirkungen.

Es wurde nun gefunden, daß die neuen Pyridazinone der allgemeinen Formel

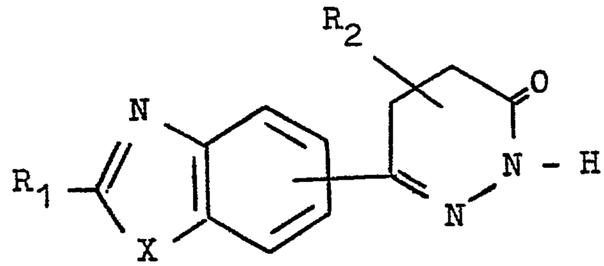

,(I)

in der

X ein Sauerstoff- oder Schwefelatom,

$R_1$ einen über ein Stickstoffatom gebundenen gesättigten 5- bis 7-gliedrigen Alkyleniminoring oder einen über ein Kohlenstoffatom gebundenen 5- bis 7-gliedrigen gesättigten Imino-, N-Alkyl-imino-, N-Phenylalkyl-imino-, N-Alkanoyl-imino- oder N-Alkoxycarbonyl-imino-alkylenring, wobei die vorstehend erwähnten Ringe im Kohlenstoffgerüst durch ein oder zwei Alkylgruppen substituiert und eine Methylengruppe in 4-Stellung eines 6- oder 7-gliedrigen Ringes durch ein Sauerstoff- oder Schwefelatom, eine Sulfinyl-, Sulfonyl-, Imino-, Alkylimino-, Phenylalkylimino-, Alkoxycarbonylimino- oder Alkanoyliminogruppe ersetzt sein kann, einen gegebenenfalls durch eine oder zwei Alkylgruppen über ein Stickstoffatom gebundenen gesättigten 5- bis 7-gliedrigen Alkyleniminoring, in dem eine $-CH_2CH_2$-Gruppe durch eine –NH–CO–Gruppe ersetzt ist, wobei die CO–Gruppe dieser –NH–CO–Gruppe mit dem bereits vorhandenen N-Atom verknüpft ist, einen über ein Stickstoffatom gebundenen 5-gliedrigen heteroaromatischen Ring, welcher zusätzlich ein Sauerstoff- oder Schwefelatom, ein oder zwei Stickstoffatome, ein Sauerstoff- oder Schwefelatom und ein Stickstoffatom enthalten kann, wobei der heteroaromatische Ring durch ein oder zwei Alkylgruppen und im Falle der Pyrazole auch durch eine Hydroxygruppe und eine Alkylgruppe substituiert sein kann, einen über ein Kohlenstoffatom gebundenen 5- oder 6-gliedrigen heteroaromatischen Ring, welcher ein Sauerstoff- oder Schwefelatom, ein, zwei oder drei Stickstoffatome, ein Sauerstoff- oder Schwefelatom und ein oder zwei Stickstoffatome enthalten kann, wobei die heteroaromatischen Ringe durch ein oder zwei Alkylgruppen, durch eine Amino- oder Alkanoylaminogruppe und im Falle der Pyridine auch durch ein oder zwei Halogenatome oder durch ein Halogenatom und eine Amino- oder Morpholinogruppe substituiert sein können, und

$R_2$ ein Wasserstoffatom oder eine Alkylgruppe oder

X eine gegebenenfalls durch eine Alkyl- oder Phenylalkylgruppe substituierte Iminogruppe,

$R_1$ einen über ein Stickstoffatom gebundenen gesättigten 5- bis 7-gliedrigen Alkyleniminoring oder einen über ein Kohlenstoffatom gebundenen 5- bis 7-gliedrigen gesättigten Imino-, N-Alkyl-imino-, N-Phenylalkyl-imino-, N-Alkanoylimino- oder N-Alkoxycarbonyl-imino-alkylenring, wobei die vorstehend erwähnten Ringe im Kohlenstoffgerüst durch ein oder zwei Alkylgruppen substituiert und eine Methylengruppe in 4-Stellung eines 6- oder 7-gliedrigen Ringes durch ein Sauerstoff- oder Schwefelatom, eine Sulfinyl-, Sulfonyl-, Imino-, Alkylimino-, Phenylalkylimino-, Alkoxycarbonylimino- oder Alkanoyliminogruppe ersetzt sein kann, einen gegebenenfalls durch eine oder zwei Alkylgruppen über ein Stickstoffatom gebundenen gesättigten 5- bis 7-gliedrigen Alkyleniminoring, in dem eine $-CH_2CH_2$–Gruppe durch eine –NH–CO–Gruppe ersetzt ist, wobei die CO–Gruppe dieser –NH–CO–Gruppe mit dem bereits vorhandenen N-Atom verknüpft ist, und

$R_2$ ein Wasserstoffatom oder eine Alkylgruppe, wobei alle vorstehend erwähnten Alkyl-, Alkanoyl- oder Alkoxygruppen jeweils 1 bis 3 Kohlenstoffatome enthalten können, oder

X eine Iminogruppe,

$R_1$ eine 1-Imidazolyl-, 1-Pyrazolyl-, 3-Methyl-1-pyrazolyl-, 3-Hydroxy-4-methyl-2-pyrazolyl- oder 1-[1.2.4]Triazolylgruppe und

$R_2$ eine Methylgruppe in 5-Stellung oder

X eine Iminogruppe,

$R_1$ eine 2-Chlor-6-morpholino-4-pyridylgruppe und

$R_2$ ein Wasserstoffatom bedeuten,

welche sich von den literaturbekannten Pyridazinonen durch den über ein Stickstoffatom oder über ein Kohlenstoffatom gebundenen gesättigten oder aromatischen heterocyclischen Rest in 2-Stellung unterscheiden, und deren Säureadditionssalze, insbesondere deren physiologisch verträgliche Säureadditionssalze mit anorganischen oder organischen Säuren, überlegene pharmakologische Eigenschaften aufweisen, insbesondere antithrombotische und cardiovasculäre Eigenschaften.

Für die bei der Definition der Reste X, $R_1$ und $R_2$ eingangs erwähnten Bedeutungen kommt beispielsweise

für X die des Sauerstoff- oder Schwefelatoms, der Imino-, Methylimino-, Ethylimino-, n-Propylimino-, Benzylimino-, 1-Phenylethylimino-, 2-Phenylethylimino- oder 3-Phenylpropyliminogruppe,

für $R_1$ die der 1-Pyrrolidinyl-, Piperidino-, 1-Hexahydroazepinyl-, 2-Methyl-1-pyrrolidinyl-, 3-Methyl-1-pyrrolidinyl-, 2-Methyl-piperidino-, 2-n-Propylpiperidino-, 3-Methyl-piperidino-, 4-Methyl-piperidino-, 2-Ethyl-piperidino-, 4-Ethyl-piperidino-, 4-Isopropylpiperidino-, 2,6-Dimethyl-piperidino-, 3,5-Dimethylpiperidino-, 1-Piperazinyl-, 4-Methyl-1-piperazinyl-, 4-Ethyl-1-piperazinyl-, 4-n-Propyl-1-piperazinyl-, 4-Benzyl-1-piperazinyl-, 4-Formyl-1-piperazinyl-, 4-Acety-1-piperazinyl-, 4-Propionyl-1-piperazinyl-, 4-Methoxycarbonyl-1-piperazinyl-, 4-Ethoxycarbonyl-1-piperazinyl-, 2-Pyrrolidinyl-, 3-Pyrrolidinyl-, 2-Piperidino-, 3-Piperidino-, 4-Piperidino-, N-Methyl-2-pyrrolidinyl-, N-Methyl-3-pyrrolidinyl-, N-Methyl-4-piperidino-, N-Ethyl-4-piperidino-, N-Isopropyl-4-piperidino-, N-Formyl-4-piperidino-, N-Acetyl-4-piperidino-, N-Propionyl-4-piperidino-, Morpholino-, Thiomorpholino-, 1-Oxido-4-thiomorpholino-, 1,1-Dioxido-4-thiomorpholino-, Imidazolin-2-on-1-yl-, 2-Methyl-2-imidazolin-1-yl-, Imidazolidin-2-on-4-yl-, 2-Methyl-4, 5-dihydro-imidazol-1-yl-, Imidazolin-2-on-4-yl-, Tetrahydrofuran-2-yl-, 4-Methyl-2-piperazinyl-, 4-Ethyl-2-piperazinyl-, 4-n-Propyl-2-piperazinyl-, 1,4-Dimethyl-2-piperazinyl-, 1,4-Diethyl-2-piperazinyl-, 1-Ethyl-4-methyl-2-piperazinyl-, 1-Pyrrolyl-, 2-Pyrrolyl-, 3-Pyrrolyl-, N-Methyl-2-pyrrolyl-, N-Ethyl-2-pyrrolyl-, N-Methyl-3-pyrrolyl-, N-Ethyl-3-pyrrolyl-, 2-Thienyl-, 3-Thienyl-, 2-Pyridyl-, 3-Pyridyl-, 4-Pyridyl-, 2-Pyrazinyl-, 1-Imidazolyl-, 2-Methyl-1-imidazolyl-, 4-Methyl-1-imidazolyl-, 2-Ethyl-1-imidazolyl-, 4-Ethyl-1-imidazolyl-, 2-Amino-5-pyridyl-, 2-Formylamino-5-pyridyl-, 2-Acetylamino-5-pyridyl-, 2-Propionylamino-5-pyridyl-, 2,6-Dichlor-4-pyridyl-, 2-Chlor-6-amino-4-pyridyl-, 2-Chlor-6-morpholino-4-pyridyl-, 2,6-Dichlor-3-pyridyl-, 2-Chlor-6-morpholino-3-pyridyl-, 1-Pyrazolyl-, 4-Methyl-1-pyrazolyl-, 3-Hydroxy-4-methyl-2-pyrazolyl-, 5-Oxazolyl-, 4-Methyl-5-oxazolyl-, 4-Pyrazolyl-, 3-Amino-4-pyrazolyl-, 1-[1.2.3]Triazolyl-, 1-[1.2.4]Triazolyl-, 2-Hydroxy-1-imidazolyl-, 4-Hydroxy-1-imidazolyl-, 4-Methyl-1-imidazolyl-, 2-Imidazolyl-, Imidazolidin-2-on-1-yl-, 2-Pyrimidyl-, 5-Pyrimidyl-, 4-Pyrimidyl-, 5-Thiazolyl-, 2-Thiazolyl-, 2-Oxazolyl- oder Triazol-3-yl-gruppe in Betracht.

Bevorzugte Verbindungen der obigen allgemeinen Formel I sind jedoch diejenigen, in denen

X ein Sauerstoff- oder Schwefelatom,

$R_1$ eine über ein Stickstoffatom gebundene Pyrrolidinyl-, Piperidino-, 1-Hexahydroazepinyl-, Morpholino-, Thiomorpholino-, 1-Oxidothiomorpholino-, 1,1-Dioxidothiomorpholino-, 1-Piperazinyl-, 4-Methyl-1-piperazinyl-, 4-Acetyl-1-piperazinyl-, 4-Carbäthoxy-1-piperazinyl-, 1-Imidazolyl-, 1-Pyrazolyl-, 1-[1.2.4]Triazolyl-, Imidazolidin-2-on-1-yl- oder 3-Hydroxy-2-pyrazolylgruppe oder eine über ein Kohlenstoffatom gebundene Piperidino-, N-Acetyl-piperidino-, Furyl-, Thienyl-, Pyrrolyl-, Pyridyl-, Amino-pyridyl-, Acetylaminopyridyl-, Pyrimidyl-, Pyrazinyl-, Oxazolyl-, Dihalogen-pyridyl- oder Halogen-morpholino-pyridylgruppe, wobei alle vorstehend erwähnten Reste im Kohlenstoffgerüst durch eine Methylgruppe substituiert sein können, und

$R_2$ ein Wasserstoffatom oder eine Methylgruppe in 5-Stellung oder

X die Iminogruppe,

$R_1$ eine über ein Stickstoffatom gebundene Pyrrolidinyl-, Piperidino-, 1-Hexahydroazepinyl-, Morpholino-, Thiomorpholino-, 1-Oxidothiomorpholino-, 1, 1-Dioxidothiomorpholino-, 1-Piperazinyl-, 4-Methyl-1-piperazinyl-, 4-Acetyl-1-piperazinyl-, 4-Carbäthoxy-1-piperazinyl- oder Imidazolidin-2-on-1-yl-gruppe oder eine über ein Kohlenstoffatom gebundene Piperidino- oder N-Acetyl-piperidinogruppe, wobei alle vorstehend erwähnten Reste im Kohlenstoffgerüst durch eine Methylgruppe substituiert sein können, und

$R_2$ ein Wasserstoffatom oder eine Methylgruppe in 5-Stellung oder

X eine Iminogruppe,

$R_1$ eine 1-Imidazolyl-, 1-Pyrazolyl-, 3-Methyl-1-pyrazolyl-, 3-Hydroxy-4-methyl-2-pyrazolyl- oder 1-[1.2.4]Triazolylgruppe und

$R_2$ eine Methylgruppe in 5-Stellung oder

X eine Iminogruppe,

$R_1$ eine 2-Chlor-6-morpholino-4-pyridylgruppe und

$R_2$ ein Wasserstoffatom bedeuten.

Besonders bevorzugte Verbindungen der obigen allgemeinen Formel I sind jedoch diejenigen, in denen

X ein Sauerstoffatom,

$R_1$ die 1-Pyrrolidinyl-, l-Imidazolyl-, 2-Furyl-, 3-Thienyl-, 1-Piperazinyl-, 4-Methyl-1-piperazinyl-, 4-Carbäthoxy-1-piperazinyl-, 4-Morpholino-, 4-Thiomorpholino-, 1-Oxido-4-thiomorpholino- oder 4-Pyridylgruppe und

$R_2$ in 5-Stellung die Methylgruppe oder

X die Iminogruppe,
$R_1$ die 1-Pyrrolidinyl-, 1-Piperazinyl-, 4-Methyl-1-piperazinyl-, 4-Carbäthoxy-1-piperazinyl-, 4-Morpholino-, 4-Thiomorpholino- oder 1-Oxido-4-thiomorpholinogruppe und
$R_2$ in 5-Stellung die Methylgruppe bedeuten.

Erfindungsgemäß erhält man die neuen Verbindungen nach folgenden Verfahren:

a) Zur Herstellung von Verbindungen der allgemeinen Formel I, in der $R_1$ einen der eingangs erwähnten über ein Kohlenstoffatom gebundenen Reste darstellt:
Cyclisierung einer gegebenenfalls im Reaktionsgemisch gebildeten Verbindung der allgemeinen Formel

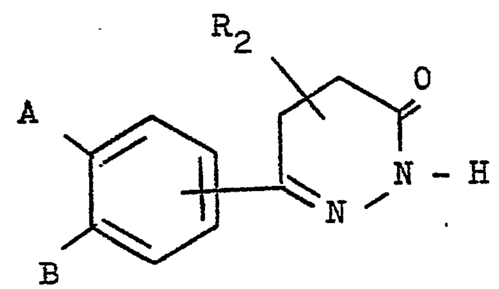

,(II)

in der
$R_2$ wie eingangs definiert ist,
einer der Reste A oder B eine

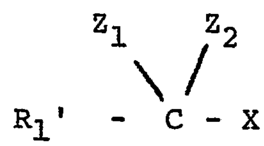 - Gruppe

und der andere der Reste A oder B eine Aminogruppe oder eine

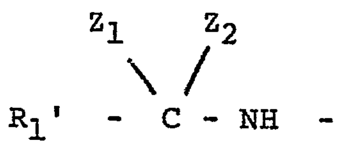 oder 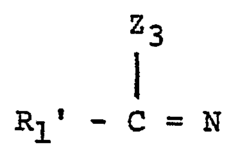 - Gruppe

bedeuten, wobei X wie eingangs definiert ist und $R_1'$ einen der für $R_1$ eingangs erwähnten über einen Kohlenstoffatom gebundenen Reste darstellt,

$Z_1$ und $Z_2$, die gleich oder verschieden sein können, nukleophile Austrittsgruppen wie gegebenenfalls substituierte Amino-, Alkoxy-, Phenylalkoxy-, Phenoxy-, Alkylmercapto-, Phenylalkylmercapto- oder Phenylthiogruppen oder

$Z_1$ und $Z_2$ zusammen ein Sauerstoff- oder Schwefelatom, eine gegebenenfalls durch eine Alkyl- oder Phenylalkylgruppe substituierte Iminogruppe, wobei die vorstehend erwähnten Alkylteile jeweils 1 bis 3 Kohlenstoffatome enthalten können, oder eine Alkylendioxigruppe mit 2 oder 3 Kohlenstoffatomen und

$Z_3$ die für $Z_1$ eingangs erwähnten Bedeutungen oder eine Imidazolylgruppe, ein Chlor-, Brom- oder Jodatom bedeuten.

Für $Z_1$ und $Z_2$ kommt beispielsweise jeweils die der Methoxy-, Ethoxy-, Propoxy-, Benzyloxy-, Methylmercapto-, Ethylmercapto-, Propylmercapto-, Phenylmercapto- oder Benzylmercaptogruppe und für $Z_3$ die des Chlor-, Brom- oder Jodatoms, der Methoxy-, Ethoxy-, Phenoxy-, Methylmercapto-, Ethylmercapto-, Phenylmercapto-, Benzylmercapto- oder Imidazolylgruppe in Betracht.

Die Umsetzung wird zweckmäßigerweise in einem Lösungsmittel oder Lösungsmittelgemisch wie Ethanol, Isopropanol, Eisessig, Tetrahydrofuran, Dioxan, Benzol, Toluol, Xylol, Chlorbenzol, Glycol, Glycolmonomethyläther, Glycoldimethyläther, Diäthylenglycoldimethylether, Dimethylformamid, Tetralin oder Sulfolan gegebenenfalls in Gegenwart eines Kondensationsmittels wie Phosphoroxychlorid, Thionylchlorid, Sulfurylchlorid, Salzsäure, Schwefelsäure, Phosphorsäure, Polyphosphorsäure, p-Toluolsulfonsäure, Eisessig, Essigsäureanhydrid, N,N'-Dicyclohexyl-carbodiimid, Carbonyldiimidazol, Kaliummethylat oder Kalium-tert.butylat bei Temperaturen zwischen 0 und 300°C, vorzugsweise jedoch bei der Siedetemperatur des Reaktionsgemisches, z.B. bei Temperaturen zwischen 50 und 285°C, durchgeführt. Die Umsetzung kann jedoch auch in der Schmelze durchgeführt werden.

Bedeutet $R_1$ einen der eingangs erwähnten über ein Kohlenstoffatom gebundenen Reste, so wird die

Umsetzung besonders vorteilhaft in einem Überschuß des für die Herstellung einer entsprechenden Verbindung der allgemeinen Formel II eingesetzten Acylierungsmittels, z.B. mit einem Überschuß des eingesetzten Nitrils, Anhydrids, Esters, Thioesters, Orthoesters, Amids, Thioamids, Halogenids oder Methojodids durchgeführt.

b) Umsetzung einer Carbonsäure der allgemeinen Formel

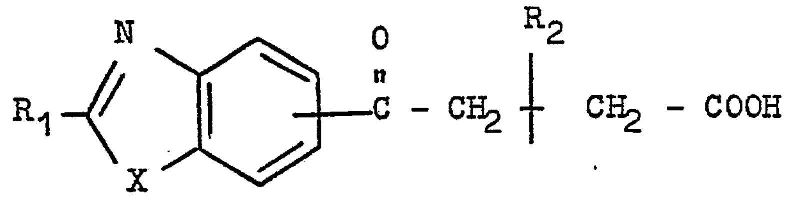

,(III)

in der

$R_1$ und $R_2$ wie eingangs definiert sind, oder deren reaktionsfähige Säurederivate wie deren Ester, Amide oder Halogenide mit Hydrazin.

Die Umsetzung wird zweckmäßigerweise in einem Lösungsmittel wie Methanol, Äthanol, Isopropanol, Eisessig, Propionsäure und/oder in einem Überschuß von Hydrazin bzw. Hydrazinhydrat bei Temperaturen zwischen 0 und 200°C, z.B. bei Temperaturen zwischen 20 und 150°C, vorzugsweise jedoch bei der Siedetemperatur des Reaktionsgemisches, und gegebenenfalls in Gegenwart einer Säure wie Schwefelsäure oder p-Toluolsulfonsäure als Kondensationsmittel durchgeführt. Die Umsetzung kann jedoch auch ohne Lösungsmittel durchgeführt werden.

c) Zur Herstellung von Verbindungen der allgemeinen Formel I, in der $R_1$ einen der eingangs erwähnten über ein Stickstoffatom gebundenen Reste darstellt:

Umsetzung einer Verbindung der allgemeinen Formel

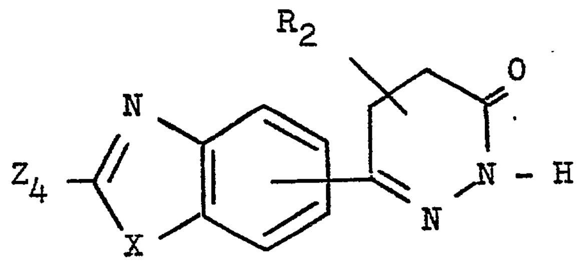

,(IV)

in der

$R_2$ und X wie eingangs definiert sind und

$Z^4$ eine nukleophil austauschbare Gruppe wie ein Halogenatom, eine Sulfogruppe oder eine substituierte Mercaptogruppe, z.B. ein Chlor- oder Bromatom, eine Sulfo-, Methylthio-, Ethylthio-, Phenylthio-, 2-Nitrophenylthio-, 4-Nitrophenylthio-, 2,4-Dinitrophenylthio-, Pyridin-4-thio- oder Pyridin-2-thiogruppe bedeutet, mit einem Amin der allgemeinen Formel

$$H–R1'', \qquad (V)$$

in der

$R_1''$ einen der für $R_1$ eingangs erwähnten über ein Stickstoffatom gebundenen Rest darstellt.

Die Umsetzung wird in der Schmelze oder in einem geeigneten Lösungsmittel wie Tetrahydrofuran, Dioxan, Dimethylformamid oder Dimethylsulfoxid gegebenenfalls in Gegenwart eines säurebindenden Mittels wie Kaliumcarbonat oder Pyridin bei erhöhten Temperaturen, z.B. bei Temperaturen zwischen 50 und 200°C, vorzugsweise jedoch bei Temperaturen zwischen 100 und 180°C, durchgeführt.

d) Zur Herstellung von Verbindungen der allgemeinen Formel I, in der X ein Sauerstoffatom oder eine gegebenenfalls durch eine Alkyl- oder Phenylalkylgruppe substituierte Iminogruppe darstellt:

Hydrierung einer Verbindung der allgemeinen Formel

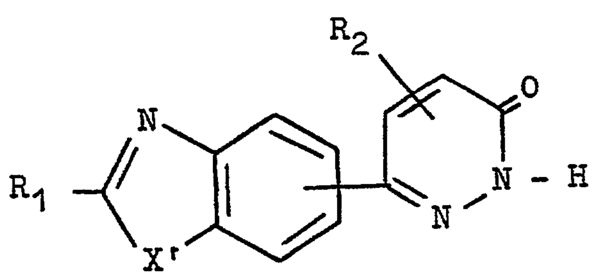

,(VI)

in der

$R_1$ und $R_2$ wie eingangs definiert sind und X' ein Sauerstoffatom oder eine gegebenenfalls durch eine Alkyl- oder Phenylalkylgruppe substituierte Iminogruppe, wobei der Alkylteil jeweils 1 bis 3 Kohlenstoffatome enthalten kann, bedeutet.

Die katalytische Hydrierung wird in einem Lösungsmittel wie Methanol, Ethanol, Essigsäureäthylester, Eisessig oder Dimethylformamid mit Wasserstoff in Gegenwart eines Hydrierungskatalysators wie Raney-Nickel, Platin oder Palladium/Kohle bei Temperaturen zwischen 0 und 75°C, vorzugsweise jedoch bei Raumtemperatur, und bei einem Wasserstoffdruck von 1 bis 5 bar durchgeführt.

Erhält man erfindungsgemäß eine Verbindung der allgemeinen Formel I, in der $R_1$ eine Imino- und/oder Aminogruppe enthält, so kann diese mittels Alkanoylierung in eine entsprechende Verbindung der allgemeinen Formel I, in der $R_1$ eine Alkanoylimino- und/oder Alkanoylaminogruppe enthält, übergeführt werden oder

eine Verbindung der allgemeinen Formel I, in der $R_1$ einen über ein Kohlenstoffatom gebundenen durch Halogenatome substituierten Pyridinrest darstellt, so kann diese mittels katalytischer Enthalogenierung und Hydrierung in eine Verbindung der allgemeinen Formel I, in der $R_1$ einen über ein Kohlenstoffatom gebundenen Piperidinorest darstellt, übergeführt werden, oder

eine Verbindung der allgemeinen Formel I, in der $R_1$ einen über ein Kohlenstoffatom gebundenen durch ein oder zwei Halogenatome substituierten Pyridinrest darstellt, so kann diese durch Halogenaustausch in eine entsprechende Verbindung der allgemeinen Formel I, in der $R_1$ einen über ein Kohlenstoffatom gebundenen Pyridinrest darstellt, der durch eine Amino- oder Morpholinogruppe und gegebenenfalls durch ein Halogenatom substituiert ist, übergeführt werden.

Die nachträgliche Alkanoylierung wird zweckmäßigerweise in einem Lösungsmittel wie Methylenchlorid, Chloroform, Diethylether, Tetrahydrofuran, Dioxan, Benzol, Acetonitril oder Dimethylformamid mit einem entsprechenden Ester, Anhydrid oder Säurehalogenid gegebenenfalls in Gegenwart eines säurebindenden Mittels wie Natriumcarbonat oder Pyridin oder mit einer entsprechenden Alkansäure in Gegenwart eines die Säure aktivierenden Mittels oder wasserentziehenden Mittels, z.B. in Gegenwart von Chlorameisensäureäthylester, Thionylchlorid, Phosphortrichlorid, N,N'-Dicyclohexylcarbodiimid, N,N'-Carbonyldiimidazol oder N,N'-Thionyldiimidazol bei Temperaturen zwischen 0 und 200°C, vorzugsweise jedoch bei Temperaturen zwischen 20 und 100°C, durchgeführt.

Die nachträgliche Enthalogenierung und Hydrierung wird in einem Lösungsmittel wie Methanol, Ethanol, Essigsäureäthylester, Eisessig oder Dimethylformamid mit Wasserstoff in Gegenwart eines Hydrierungskatalysators wie Platin oder Palladium/Kohle bei Temperaturen zwischen 0 und 75°C, vorzugsweise jedoch bei Raumtemperatur, und bei einem Wasserstoffdruck von 1 bis 5 bar durchgeführt.

Der nachträgliche Halogenaustausch wird mit Ammoniak oder Morpholin zweckmäßigerweise in einem Lösungsmittel wie Dioxan oder Dimethylformamid gegebenenfalls in einem Druckgefäß bei Temperaturen zwischen 50 und 150°C durchgeführt.

Die so erhaltenen Verbindungen der allgemeinen Formel I können aufgrund ihres optisch aktiven Kohlenstoffatoms in Position 4 oder 5 des Pyridazinon-Ringes, sofern $R_2$ eine Alkylgruppe darstellt, in ihre optisch aktiven Antipoden mittels Racematspaltung aufgetrennt werden.

Die Racematspaltung wird zweckmäßigerweise durch fraktionierte Kristallisation der entsprechenden Salze mit optisch aktiven Säuren, wie Weinsäure, Dibenzoylweinsäure, Äpfelsäure, Camphersäure oder Camphersulfonsäure oder durch Chromatographie an optisch aktiven Adsorbentien durchgeführt.

Des weiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II bis VI erhält man nach literaturbekannten Methoden bzw. sind literaturbekannt.

So erhält man beispielsweise eine entsprechende o-Diaminoverbindung der allgemeinen Formel II durch Umsetzung einer entsprechenden 3-(3-Nitro-4-acylamino-benzoyl)-propionsäure mit Hydrazin und anschließende Reduktion der Nitrogruppe (siehe US-A 4 026 891), eine entsprechende o-Hydroxyacylamino- oder o-Mercapto-acylaminoverbindung der allgemeinen Formel II durch Umsetzung einer entsprechend substituierten 3-Benzoyl-propionsäure mit Hydrazin, gegebenenfalls anschließende Reduktion der Nitrogruppe und anschließende Acylierung der so erhaltenen Aminoverbindung.

Eine als Ausgangsstoff verwendete Verbindung der allgemeinen Formel III erhält man durch Umsetzung eines entsprechenden p,α-Dihalogen-acetophenons mit einem entsprechenden Malonester. Das so erhaltene entsprechend substituierte 3-Benzoyl-propionsäurederivat wird anschließend verseift, decarboxyliert, nitriert, das Halogenatom durch eine Amino-, Hydroxy- oder Mercaptogruppe ersetzt, die Nitrogruppe reduziert und die so erhaltene Aminoverbindung nach Umsetzung mit einem entsprechenden Carbonsäurederivat zu der gewünschten Verbindung der allgemeinen Formel III umgesetzt.

Eine als Ausgangsstoff verwendete Verbindung der allgemeinen Formel IV erhält man beispielsweise durch Ringschluß eines entsprechenden Thioharnstoffs oder durch Umsetzung einer entsprechenden o-Amino-hydroxy-Verbindung mit Kaliumethylxanthogenat und anschließender Umsetzung des so erhaltenen 2-Mercaptobenzoxazols mit einem entsprechenden Elektrophil, z.B. mit einem Alkylhalogenid oder einem Nitrohalogenbenzol.

Eine als Ausgangsstoff verwendete Verbindung der allgemeinen Formel VI erhält man durch Umsetzung eines entsprechenden Acrylsäurederivates mit Hydrazin.

Die neuen Verbindungen der allgemeinen Formel I, deren optisch aktiven Antipoden und deren Säureadditionssalze, insbesondere deren physiologisch verträgliche Säureadditionssalze mit anorganischen oder organischen Säuren weisen, wie bereits eingangs erwähnt, wertvolle pharmakologische Eigenschaften auf, insbesondere cardiovasculäre Wirkungen, nämlich eine cardiotonische und/oder blutdrucksenkende, und eine antithrombotische Wirkung.

Beispielsweise wurden die Verbindungen

A = 5-Methyl-6-[2-(1-imidazolyl)-benzoxazol-5-yl]-4,5-dihydro-3(2H)-pyridazinon,
B = 5-Methyl-6-[2-(1-piperazinyl)-benzoxazol-6-yl]-4,5-dihydro-3(2H)-pyridazinon,
C = 5-Methyl-6-[2-(4-carbäthoxy-1-piperazinyl)-benzoxazol-6-yl]-4,5-dihydro-3(2H)-pyridazinon,
D = 5-Methyl-6-[2-(1-pyrrolidinyl)-benzimidazol-5-yl]-4,5-dihydro-3(2H)-pyridazinon,
E = 5-Methyl-6-[2-(4-thiomorpholino)-benzimidazol-5-yl]-4,5-dihydro-3(2H)-pyridazinon und
F = 5-Methyl-6-[2-(1-imidazolyl)-benzimidazol-5-yl]-4,5-dihydro-3(2H)-pyridazinon

auf ihre biologischen Eigenschaften wie folgt untersucht:

1. Bestimmung der Thrombozytenaggregation nach Born und Cross (J. Physiol. 170, 397 (1964)):

Die Thrombozytenaggregation wurde in plättchenreichem Plasma gesunder Versuchspersonen gemessen. Hierbei wurde der Verlauf der Abnahme der optischen Dichte nach Zugabe von handelsüblichem Collagen der Firma Sigma, St. Louis/USA, welches 1 mg Collagen-Fibrillen pro ml enthält, photometrisch gemessen und registriert. Aus dem Neigungswinkel der Dichtekurve wurde auf die Aggregationsgeschwindigkeit ($V_{max}$) geschlossen. Der Punkt der Kurve, bei dem die größte Lichtdurchlässigkeit vorlag, diente zur Berechnung der "optical density" (O.D.). Zur maximalen Aggregationsauslösung werden ca. 0,01 ml der Collagenlösung zu 1 ml plättchenreichem Plasma gegeben.

Die nachfolgende Tabelle enthält den gefundenen Wert:

Tabelle I

| Verbindung | $EC_{50}$ |
|---|---|
| A | $3,1 \times 10^{-8}$ Mol/l |

2. Bestimmung der blutdrucksenkenden und positiv inotropen Wirkung an der narkotisierten Katze:

Die Untersuchungen wurden an Katzen durchgeführt, die mit Pentobarbital-Natrium (40 mg/kg i.p.) narkotisiert waren.

Die Tiere atmeten spontan. Der arterielle Blutdruck wurde in der Aorta abdominalis mit einem Statham-Druckwandler (p 23 Dc) gemessen. Für die Erfassung der positiv inotropen Wirkung wurde mit einem Kathetertipmanometer (Milliar PC-350 A) der Druck in der linken Herzkammer gemessen. Daraus wurde der Kontraktilitätsparameter $dp/dt_{max}$ mittels eines Analogdifferenzierers gewonnen. Die zu untersuchende Substanz wurden in eine Vena femoralis injiziert. Als Lösungsmittel diente Polydiol 200. Die Substanz wurde an 3 Katzen geprüft, Dosis 0,1 i.v.

Die nachfolgende Tabelle enthält die Mittelwerte:

| Substanz | Dosis mg/kg i.v. | Blutdruckänderung in mmHg | Zunahme von dp/dt in % | Wirkungsdauer (Halbwertszeit) |
|---|---|---|---|---|
| A | 0,1 | –43/–45 | +49 | 25 Minuten |
| B | 0,1 | –45/–43 | +88 | 12 Minuten |
| C | 0,1 | –45/–50 | +66 | 12 Minuten |
| D | 0,1 | –13/–17 | +68 | 13 Minuten |
| E | 0,1 | +2/–12 | +65 | 10 Minuten |
| F | 0,1 | –35/–20 | +81 | 8 Minuten |

In diesem Zusammenhang sei darauf hingewiesen, daß bei den biologischen Untersuchungen keine toxischen Nebenwirkungen der Substanzen beobachtet wurden.

Aufgrund ihrer pharmakologischen Eigenschaften eignen sich die erfindungsgemäß hergestellten Verbindungen der allgemeinen Formel I und deren optisch aktive Antipoden sowie deren physiologisch

verträgliche Säureadditionssalze mit anorganischen oder organischen Säuren zur Behandlung der chronischen Herzinsuffizienz oder der Angina Pectoris und/oder zur Prophylaxe arterieller Thromboembolien und arterieller Verschlußkrankheiten.

Hierzu lassen sich die neuen Verbindungen, gegebenenfalls in Kombination mit anderen Wirksubstanzen, in die üblichen pharmazeutischen Anwendungsformen wie Tabletten, Dragées, Pulver, Suspensionen, Suppositorien oder Ampullen einarbeiten. Die Einzeldosis beträgt hierbei beim Erwachsenen 1–4 x täglich bei intravenöser Applikation 1–50 mg, vorzugsweise 2 bis 40 mg, und bei oraler Applikation 5–150 mg, vorzugsweise 5 bis 100 mg.

Die nachfolgenden Beispiele sollen die Erfindung näher erläutern:

Beispiel 1

5-Methyl-6-[2-(3-pyridyl)-benzoxazol-5-yl]-4,5-dihydro-3(2H)-pyridazinon

2 g (6,0 mMol) N-[2-Hydroxy-5-(5-methyl-4,5-dihydro-3(2H)-pyridazinon-6-yl)-phenyl]-pyridin-3-carbonsäureamid werden in 40 ml Eisessig suspendiert und 20 Stunden lang in einer Stahlbombe mit Glaseinsatz auf 150°C erhitzt. Danach wird die Essigsäure abdestilliert, der Rückstand mit Wasser verrührt, dann abgesaugt und getrocknet. Die weitere Reinigung erfolgt durch Chromatographie über Kieselgel (Elutionsmittel: Dichlormethan + 6% Ethanol).

Ausbeute: 38,3% der Theorie,
Schmelzpunkt: 223–225°C

| | | | | | | |
|---|---|---|---|---|---|---|
| Ber.: | C | 66,66 | H | 4,61 | N | 18,29 |
| Gef.: | | 66,53 | | 4,35 | | 18,20 |

Beispiel 2

5-Methyl-6-[2-(4-pyridyl)-benzoxazol-5-yl]-4,5-dihydro-3(2H)-pyridazinon

Hergestellt aus N-[2-Hydroxy-5-(5-methyl-4,5-dihydro-3(2H)-pyridazinon-6-yl)-phenyl]-pyridin-4-carbonsäureamid analog Beispiel 1.

Ausbeute: 39,9% der Theorie,
Schmelzpunkt: 238–240°C

| | | | | | | |
|---|---|---|---|---|---|---|
| Ber.: | C | 66,66 | H | 4,61 | N | 18,29 |
| Gef.: | | 67,00 | | 4,73 | | 18,65 |

Beispiel 3

5-Methyl-6-[2-(2-acetamino-5-pyridyl)-benzoxazol-5-yl]-4,5-dihydro-3(2H)-pyridazinon

Hergestellt aus N-[2-Hydroxy-5-(5-methyl-4,5-dihydro-3(2H)-pyridazinon-6-yl)-phenyl]-2-acetamino-pyridin-5-carbonsäureamid analog Beispiel 1.

Ausbeute: 20,7% der Theorie,
Schmelzpunkt: 299–300°C

| | | | | | | |
|---|---|---|---|---|---|---|
| Ber.: | C | 62,80 | H | 4,72 | N | 19,27 |
| Gef.: | | 62,90 | | 5,01 | | 18,98 |

Beispiel 4

5-Methyl-6-[2-(2-furyl)-benzoxazol-5-yl]-4,5-dihydro-3(2H)-pyridazinon

Hergestellt aus N-[2-Hydroxy-5-(5-methyl-4,5-dihydro-3(2H)-pyridazinon-6-yl)-phenyl]-furan-2-carbonsäureamid analog Beispiel 1.

Ausbeute: 22,2% der Theorie,
Schmelzpunkt: 204–205°C

| | | | | | | |
|---|---|---|---|---|---|---|
| Ber.: | C | 65,08 | H | 4,44 | N | 14,23 |
| Gef.: | | 64,79 | | 4,44 | | 14,27 |

Beispiel 5

5-Methyl-6-[2-(2-thienyl)-benzoxazol-5-yl]-4,5-dihydro-3(2H)-pyridazinon

Hergestellt aus N-[2-Hydroxy-5-(5-methyl-4,5-dihydro-3(2H)-pyridazinon-6-yl)-phenyl]-thiophen-2-carbonsäureamid analog Beispiel 1.
Ausbeute: 50,5% der Theorie,
Schmelzpunkt: 214–215°C

| | | | | | | | | |
|---|---|---|---|---|---|---|---|---|
| Ber.: | C | 61,71 | H | 4,21 | N | 13,50 | S | 10,30 |
| Gef.: | | 61,46 | | 4,25 | | 13,31 | | 10,37 |

Beispiel 6

5-Methyl-6-[2-(3-thienyl)-benzoxazol-5-yl]-4,5-dihydro-3(2H)-pyridazinon

Hergestellt aus N-[2-Hydroxy-5-(5-methyl-4,5-dihydro-3(2H)-pyridazinon-6-yl)-phenyl]-thiophen-3-carbonsäureamid analog Beispiel 1.
Ausbeute: 22,3% der Theorie,
Schmelzpunkt: 222–224°C

| | | | | | | | | |
|---|---|---|---|---|---|---|---|---|
| Ber.: | C | 61,71 | H | 4,21 | N | 13,50 | S | 10,30 |
| Gef.: | | 61,37 | | 4,12 | | 13,50 | | 10,60 |

Beispiel 7

6-[2-(4-Methyl-oxazol-5-yl)-benzoxazol-5-yl]-4,5-dihydro-3(2H)-pyridazinon

Hergestellt aus N-[2-Hydroxy-5-(4,5-dihydro-3(2H)-pyridazinon-6-yl)-phenyl]-4-methyl-oxazol-5-carbonsäureamid analog Beispiel 1.
Ausbeute: 12,7% der Theorie,
Schmelzpunkt: 238–240°C

| | | | | | | |
|---|---|---|---|---|---|---|
| Ber.: | C | 60,80 | H | 4,08 | N | 18,91 |
| Gef.: | | 60,98 | | 4,18 | | 18,90 |

Beispiel 8

6-[2-(3-Thienyl)-benzoxazol-5-yl]-4,5-dihydro-3(2H)-pyridazinon

Hergestellt aus N-[2-Hydroxy-5-(4,5-dihydro-3(2H)-pyridazinon-6-yl)-phenyl]-thiophen-3-carbonsäureamid analog Beispiel 1.
Ausbeute: 15% der Theorie,
Schmelzpunkt: 262–263°C

| | | | | | | | | |
|---|---|---|---|---|---|---|---|---|
| Ber.: | C | 60,60 | H | 3,73 | N | 14,14 | S | 10,79 |
| Gef.: | | 60,56 | | 3,54 | | 14,00 | | 10,93 |

Beispiel 9

5-Methyl-6-[2-(2-thienyl)-benzthiazol-5-yl]-4,5-dihydro-3(2H)-pyridazinon

1,7 g (4,9 mMol) N-[2-Mercapto-5-(5-methyl-4,5-dihydro-3(2H)-pyridazinon-6-yl)-phenyl]-thiophen-2-carbonsäureamid werden geschmolzen, bis keine Gasentwicklung mehr zu beobachten ist. Durch Säulenchromatographie über 200 g Kieselgel (Elutionsmittel: Dichlormethan + 5% Ethanol) wird das so erhaltene Produkt gereinigt.
Ausbeute: 11% der Theorie,
Schmelzpunkt: 203–204°C

$C_{16}H_{13}N_3OS_2$ (327,4)

| | | | | | | | | |
|---|---|---|---|---|---|---|---|---|
| Ber.: | C | 58,69 | H | 4,00 | N | 12,83 | S | 19,59 |
| Gef.: | | 58,33 | | 4,12 | | 13,01 | | 19,44 |

Beispiel 10

5-Methyl-6-[2-(2-furyl)-benzthiazol-5-yl]-4,5-dihydro-3(2H)-pyridazinon

Hergestellt aus N-[2-Mercapto-5-(5-methyl-4,5-dihydro-3(2H)-pyridazinon-6-yl]-furan-2-carbonsäureamid analog Beispiel 9.
Ausbeute: 28% der Theorie,
Schmelzpunkt: 223–224°C

| | | | | | | | | |
|---|---|---|---|---|---|---|---|---|
| Ber.: | C | 61,72 | H | 4,21 | N | 13,50 | S | 10,30 |
| Gef.: | | 61,90 | | 4,21 | | 13,40 | | 10,43 |

Beispiel 11

5-Methyl-6-[2-(1-piperazinyl)-benzoxazol-5-yl]-4,5-dihydro-3(2H)-pyridazinon

1,5 g (5,4 mMol) 5-Methyl-6-(2-methylthio-benzoxazol-5-yl)-4,5-dihydro-3(2H)-pyridazinon werden mit 10 g Piperazin gemischt und 2 Stunden lang auf 160°C erhitzt. Danach wird das Reaktionsgemisch mit ca. 300 ml Wasser verrührt, das ungelöste Produkt abgesaugt und mit Wasser gewaschen. Die Reinigung erfolgt durch Säulenchromatographie (basisches Aluminiumoxid, Elutionsmittel: Methylenchlorid + 2% Ethanol).
Ausbeute: 44,4% der Theorie,
Schmelzpunkt: 215–216°C

$C_{16}H_{19}N_5O_2$ (313,4)

| | | | | | | |
|---|---|---|---|---|---|---|
| Ber.: | C | 61,33 | H | 6,11 | N | 22,35 |
| Gef.: | | 61,15 | | 6,12 | | 22,00 |

Beispiel 12

5-Methyl-6-[2-(1-pyrrolidinyl)-benzoxazol-5-yl]-4,5-dihydro-3(2H)-pyridazinon

Hergestellt aus 5-Methyl-6-(2-methylthio-benzoxazol-5-yl)-4,5-dihydro-3(2H)-pyridazinon und Pyrrolidin analog Beispiel 11.
Ausbeute: 59,4% der Theorie,
Schmelzpunkt: 230–231°C

| | | | | | | |
|---|---|---|---|---|---|---|
| Ber.: | C | 64,41 | H | 6,08 | N | 18,78 |
| Gef.: | | 64,70 | | 6,16 | | 18,86 |

Beispiel 13

5-Methyl-6-[2-(1-imidazolyl)-benzoxazol-5-yl]-4,5-dihydro-3(2H)-pyridazinon

Hergestellt aus 5-Methyl-6-(2-methylthio-benzoxazol-5-yl)-4,5-dihydro-3(2H)-pyridazinon und Imidazol analog Beispiel 11.
Ausbeute: 31,4% der Theorie,
Schmelzpunkt: 236–238°C

| | | | | | | |
|---|---|---|---|---|---|---|
| Ber.: | C | 61,01 | H | 4,44 | N | 23,72 |
| Gef.: | | 60,76 | | 4,62 | | 24,01 |

Beispiel 14

5-Methyl-6-[2-(4-methyl-1-piperazinyl)-benzoxazol-5-yl]-4,5-dihydro-3(2H)-pyridazinon

Hergestellt aus 5-Methyl-6-(2-methylthio-benzoxazol-5-yl)-4,5-dihydro-3(2H)-pyridazinon und 1-Methyl-piperazin analog Beispiel 11.

Ausbeute: 58,8% der Theorie,
Schmelzpunkt: 230–231°C

| | | | | | | |
|---|---|---|---|---|---|---|
| Ber.: | C | 62,37 | H | 6,47 | N | 21,39 |
| Gef.: | | 62,56 | | 6,53 | | 21,44 |

Beispiel 15

5-Methyl-6-[2-(2-methyl-1-imidazolyl)-benzoxazol-5-yl]-4,5-dihydro-3(2H)-pyridazinon

Hergestellt aus 5-Methyl-6-(2-methylthio-benzoxazol-5-yl)-4,5-dihydro-3(2H)-pyridazinon und 2-Methyl-imidazol in Dimethylsulfoxid durch 3stündiges Erhitzen auf 150°C analog Beispiel 11.
Ausbeute: 18,8% der Theorie,
Schmelzpunkt: 214–216°C

| | | | | | | |
|---|---|---|---|---|---|---|
| Ber.: | C | 62,13 | H | 4,89 | N | 22,64 |
| Gef.: | | 62,48 | | 4,87 | | 22,82 |

Beispiel 16

6-[2-(2-Methyl-1-imidazolyl)-benzoxazol-5-yl]-4,5-dihydro-3(2H)-pyridazinon

Hergestellt aus 6-(2-Methylthio-benzoxazol-5-yl)-4,5-dihydro-3(2H)-pyridazinon und 2-Methyl-imidazol analog Beispiel 11.
Ausbeute: 54,2% der Theorie,
Schmelzpunkt: 242–243°C

| | | | | | | |
|---|---|---|---|---|---|---|
| Ber.: | C | 61,01 | H | 4,44 | N | 23,72 |
| Gef.: | | 61,00 | | 4,38 | | 23,78 |

Beispiel 17

5-Methyl-6-[2-(4-methyl-1-pyrazolyl)-benzoxazol-5-yl]-4,5-dihydro-3(2H)-pyridazinon

Hergestellt aus 5-Methyl-6-(2-methylthio-benzoxazol-5-yl)-4,5-dihydro-3(2H)-pyridazinon und 4-Methyl-pyrazol analog Beispiel 11.
Ausbeute: 16,6% der Theorie,
Schmelzpunkt: 255–257°C

| | | | | | | |
|---|---|---|---|---|---|---|
| Ber.: | C | 62,13 | H | 4,89 | N | 22,64 |
| Gef.: | | 61,90 | | 5,00 | | 22,58 |

Beispiel 18

6-[2-(N-Acetyl-4-piperidino)-benzimidazol-5-yl]-4,5-dihydro-3(2H)-pyridazinon-hydrochlorid

450 mg 6-[2-(4-Piperidino)-benzimidazol-5-yl]-4,5-dihydro-3(2H)-pyridazinon werden in 50 ml Essigester und 15 ml Eisessig suspendiert. Hierzu gibt man 15 ml Acetanhydrid und erhitzt 2 Stunden am Rückfluß. Das Lösungsmittel wird entfernt und der Rückstand in Äther/Aceton verrieben. Anschließend wird kurz mit Wasser erhitzt und nach Entfernen des Wassers der erhaltene Rückstand in Äthanol gelöst, mit Aktivkohle versetzt und abfiltriert. Beim Versetzen des Filtrats mit ätherischer Salzsäure erhält man das gewünschte Produkt.
Ausbeute: 210 mg (37% der Theorie),
Schmelzpunkt: ab 237°C (Aceton).

Beispiel 19

5-Methyl-6-[2-(N-acetyl-4-piperidino)-benzimidazol-5-yl]-4,5-dihydro-3(2H)-pyridazinon

Hergestellt aus 5-Methyl-6-[2-(4-piperidino)-benzimidazol-5-yl]-4,5-dihydro-3(2H)-pyridazinon und Acetanhydrid analog Beispiel 18.

Ausbeute: 16% der Theorie,
Schmelzpunkt: 245–248°C

| | | | | | | |
|---|---|---|---|---|---|---|
| Ber.: | C | 64,57 | H | 6,56 | N | 19,82 |
| Gef.: | | 64,40 | | 6,54 | | 20,00 |

Beispiel 20

6-[2-(4-piperidino)-benzimidazol-5-yl]-4,5-dihydro-3(2H)-pyridazinon-hydrochlorid

3,6 g 6-[2-(2,6-Dichlor-4-pyridyl)-benzimidazol-5-yl]-4,5-dihydro-3(2H)-pyridazinon werden in 100 ml Methanol 6 Stunden lang in einem Druckgefäß bei 50°C und 5 bar Wasserstoff in Gegenwart von Palladium/Kohle hydriert. Anschließend wird abfiltriert, das Filtrat mit Ether versetzt und die ausgefallenen Kristalle abgesaugt. Das erhaltene Rohprodukt wird in Methanol gelöst, mit Aktivkohle versetzt und abfiltriert. Beim Versetzen des Filtrats mit etherischer Salzsäure erhält man das gewünschte Produkt.
Ausbeute: 2,05 g (61,4% der Theorie),
Schmelzpunkt: sintern ab 100°C

Beispiel 21

6-[2-(2-Chlor-6-morpholino-4-pyridyl)-benzimidazol-5-yl]-4,5-dihydro-3(2H)-pyridazinon

900 mg 6-[2-(2,6-Dichlor-4-pyridyl)-benzimidazol-5-yl]-4,5-dihydro-3(2H)-pyridazinon werden in 15 ml Morpholin suspendiert und 1 Stunde auf 100°C erhitzt. Anschließend wird abgekühlt, abgesaugt, 1 x mit Wasser ausgekocht und gut getrocknet. Danach wird der erhaltene Rückstand in Dimethylformamid gelöst, mit Aktivkohle versetzt, abfiltriert und das gewünschte Produkt durch Zusatz von Petrolether ausgefällt.
Ausbeute: 300 mg (29,2% der Theorie),
Schmelzpunkt: über 300°C

Beispiel 22

5-Methyl-6-[2-(2-pyridyl)-benzoxazol-6-yl]-4,5-dihydro-3(2H)-pyridazinon

6,5 g (20 mMol) N-[2-Hydroxy-4-(5-methyl-4,5-dihydro-3(2H)-pyridazinon-6-yl)-phenyl]-pyridin-2-carbonsäureamid (hergestellt aus Pyridin-2-carbonsäure, N,N'-Carbonyldiimidazol und 2-Hydroxy-4-(5-methyl-4,5-dihydro-3(2H)-pyridazinon-6-yl)-anilin) werden bei 120°C unter Rühren in 60 g Polyphosphorsäure eingetragen und 1 Stunde bei dieser Temperatur gehalten. Nach dem Abkühlen wird mit Eiswasser verdünnt, mit Ammoniak alkalisch gestellt und mit Methylenchlorid extrahiert. Der Extrakt wird mit Wasser gewaschen, mit Natriumsulfat getrocknet und das Lösungsmittel im Vakuum abdestilliert. Der Rückstand wird mit Aceton aufgerührt, die anfallenden Kristalle abgesaugt und aus Isopropanol/Wasser umkristallisiert.
Ausbeute: 4,5 g (73,3% der Theorie),
Schmelzpunkt: 233°C

| | | | | | | |
|---|---|---|---|---|---|---|
| Ber.: | C | 66,66 | H | 4,61 | N | 18,29 |
| Gef.: | | 66,75 | | 4,76 | | 18,28 |

Beispiel 23

5-Methyl-6-[2-(3-thienyl)-benzoxazol-6-yl]-4,5-dihydro-3(2H)-pyridazinon

3,0 g (9,1 mMol) N-[2-(Hydroxy-4-(5-methyl-4,5-dihydro-3(2H)-pyridazinon-6-yl]-thiophen-3-carbonsäureamid (hergestellt aus Thiophen-3-carbonsäure, N,N'-Carbonyldiimidazol und 2-Hydroxy-4-(5-methyl-4,5-dihydro-3(2H)-pyridazinon-6-yl)-anilin und 0,5 g p-Toluolsulfonsäure werden in 50 ml Eisessig gelöst und 8 Stunden zum Rückfluß erhitzt. Der Eisessig wird dann im Vakuum abdestilliert, der Rückstand mit Wasser und Diisopropyläther aufgerührt, abgesaugt und mit Wasser und dann mit Aceton gewaschen. Das Rohprodukt kristallisiert man aus Dioxan um.
Ausbeute: 1,6 g (56,4% der Theorie),
Schmelzpunkt: 235°C

| | | | | | | | | |
|---|---|---|---|---|---|---|---|---|
| Ber.: | C | 61,72 | H | 4,21 | N | 13,50 | S | 10,30 |
| Gef.: | | 61,45 | | 4,45 | | 13,52 | | 10,14 |

Beispiel 24

5-Methyl-6-[2-(2-furyl)-benzoxazol-6-yl]-4,5-dihydro-3(2H)-pyridazinon

Hergestellt aus N-[2-Hydroxy-4-(5-methyl-4,5-dihydro-3(2H)-pyridazinon-6-yl)-phenyl]-furan-2-carbonsäureamid und Polyphosphorsäure analog Beispiel 22.
Ausbeute: 40% der Theorie,
Schmelzpunkt: 209°C

| | | | | | | | | |
|---|---|---|---|---|---|---|---|---|
| Ber.: | C | 65,08 | H | 4,44 | N | 14,23 | O | 16,25 |
| Gef.: | | 64,93 | | 4,61 | | 13,99 | | 16,47 |

Beispiel 25

5-Methyl-6-[2-(2-thienyl)-benzoxazol-6-yl]-4,5-dihydro-3(2H)-pyridazinon

Hergestellt aus N-[2-Hydroxy-4-(5-methyl-4,5-dihydro-3(2H)-pyridazinon-6-yl)-phenyl]-thiophen-3-carbonsäureamid und Polyphosphorsäure bei 130°C analog Beispiel 22.
Ausbeute: 16,1% der Theorie,
Schmelzpunkt: 222°C

| | | | | | | | | |
|---|---|---|---|---|---|---|---|---|
| Ber.: | C | 61,72 | H | 4,21 | N | 13,50 | S | 10,30 |
| Gef.: | | 61,68 | | 4,42 | | 13,50 | | 10,25 |

Beispiel 26

5-Methyl-6-[2-(3-pyridyl)-benzoxazol-6-yl]-4,5-dihydro-3(2H)-pyridazinon

Hergestellt aus N-[2-Hydroxy-4-(5-methyl-4,5-dihydro-3(2H)-pyridazinon-6-yl)-phenyl]-pyridin-3-carbonsäureamid und Polyphosphorsäure bei 150°C analog Beispiel 22.
Ausbeute: 25% der Theorie,
Schmelzpunkt: 218°C

| | | | | | | |
|---|---|---|---|---|---|---|
| Ber.: | C | 66,66 | H | 4,61 | N | 18,29 |
| Gef.: | | 66,44 | | 4,69 | | 18,15 |

Beispiel 27

5-Methyl-6-[2-(2-pyrazinyl)-benzoxazol-6-yl]-4,5-dihydro-3(2H)-pyridazinon

Hergestellt aus N-[2-Hydroxy-4-(5-methyl-4,5-dihydro-3(2H)-pyridazinon-6-yl)-phenyl]-pyrazin-2-carbonsäureamid und Polyphosphorsäure analog Beispiel 22.
Ausbeute: 21,7% der Theorie,
Schmelzpunkt: 242°C

| | | | | | | |
|---|---|---|---|---|---|---|
| Ber.: | C | 62,53 | H | 4,26 | N | 22,79 |
| Gef.: | | 62,80 | | 4,17 | | 22,60 |

Beispiel 28

5-Methyl-6-[2-(4-pyridyl)-benzoxazol-6-yl]-4,5-dihydro-3(2H)-pyridazinon

Hergestellt aus N-[2-Hydroxy-4-(5-methyl-4,5-dihydro-3(2H)-pyridazinon-6-yl)-phenyl]-pyridin-4-carbonsäureamid und Polyphosphorsäure analog Beispiel 22.
Ausbeute: 49,1% der Theorie,
Schmelzpunkt: 228°C

| | | | | | | |
|---|---|---|---|---|---|---|
| Ber.: | C | 66,66 | H | 4,61 | N | 18,29 |
| Gef.: | | 66,75 | | 4,54 | | 18,14 |

Beispiel 29

5-Methyl-6-[2-(1-acetyl-4-piperidino)-benzoxazol-6-yl]-4,5-dihydro-3(2H)-pyridazinon

Hergestellt aus N-[2-Hydroxy-4-(5-methyl-4,5-dihydro-3(2H)-pyridazinon-6-yl)-phenyl]-1-acetyl-piperidin-4-carbonsäureamid und Polyphosphorsäure analog Beispiel 22.
Ausbeute: 60,3% der Theorie,
Schmelzpunkt: 199°C

| | | | | | | |
|---|---|---|---|---|---|---|
| Ber.: | C | 64,39 | H | 6,26 | N | 15,81 |
| Gef.: | | 64,60 | | 6,20 | | 16,06 |

Beispiel 30

5-Methyl-6-[2-(4-methyl-5-oxazolyl)-benzoxazol-6-yl]-4,5-dihydro-3(2H)-pyridazinon

Hergestellt aus N-[2-Hydroxy-4-(5-methyl-4,5-dihydro-3(2H)-pyridazinon-6-yl)-phenyl]-4-methyl-oxazol-5-carbonsäureamid und Polyphosphorsäure bei 140°C analog Beispiel 22.
Ausbeute: 28,2% der Theorie,
Schmelzpunkt: 217°C

| | | | | | | |
|---|---|---|---|---|---|---|
| Ber.: | C | 61,93 | H | 4,55 | N | 18,05 |
| Gef.: | | 62,12 | | 4,70 | | 18,28 |

Beispiel 31

5-Methyl-6-[2-(5-pyrimidinyl)-benzoxazol-6-yl]-4,5-dihydro-3(2H)-pyridazinon

Hergestellt aus N-[2-Hydroxy-4-(5-methyl-4,5-dihydro-3(2H)-pyridazinon-6-yl)-phenyl]-pyrimidin-5-carbonsäureamid und Polyphosphorsäure bei 150°C analog Beispiel 22.
Ausbeute: 16,3% der Theorie,
Schmelzpunkt: 279°C

| | | | | | | |
|---|---|---|---|---|---|---|
| Ber.: | C | 62,53 | H | 4,26 | N | 22,79 |
| Gef.: | | 62,48 | | 4,27 | | 22,57 |

Beispiel 32

5-Methyl-6-[2-(2-amino-5-pyridyl)-benzoxazol-6-yl]-4,5-dihydro-3(2H)-pyridazinon

Hergestellt aus N-[2-Hydroxy-4-(5-methyl-4,5-dihydro-3(2H)-pyridazinon-6-yl)-phenyl]-6-acetamino-pyridin-3-carbonsäureamid und Polyphosphorsäure bei 150°C analog Beispiel 22.
Ausbeute: 8,2% der Theorie,
Massenspektrum: $M^+$ = 321 m/e

| | | | | | | |
|---|---|---|---|---|---|---|
| Ber.: | C | 63,54 | H | 4,71 | N | 21,79 |
| Gef.: | | 63,45 | | 4,85 | | 21,59 |

Beispiel 33

6-[2-(2-Pyridyl)-benzoxazol-6-yl]-4,5-dihydro-3(2H)-pyridazinon

Hergestellt aus N-[2-Hydroxy-4-(4,5-dihydro-3(2H)-pyridazinon-6-yl)-phenyl]-pyridin-2-carbonsäureamid und Polyphosphorsäure analog Beispiel 22.
Ausbeute: 29,1% der Theorie,
Schmelzpunkt: 282–283°C

| | | | | | | |
|---|---|---|---|---|---|---|
| Ber.: | C | 65,75 | H | 4,14 | N | 19,17 |
| Gef.: | | 66,03 | | 4,16 | | 19,33 |

Beispiel 34

6-[2-(2-Furyl)-benzoxazol-6-yl]-4,5-dihydro-3(2H)-pyridazinon

Hergestellt aus N-[2-Hydroxy-4-(4,5-dihydro-3(2H)-pyridazinon-6-yl)-phenyl]-furan-2-carbonsäureamid und Polyphosphorsäure analog Beispiel 22.
Ausbeute: 31,3% der Theorie,
Schmelzpunkt: 261–262°C

| | | | | | | |
|---|---|---|---|---|---|---|
| Ber.: | C | 64,05 | H | 3,94 | N | 14,94 |
| Gef.: | | 63,89 | | 4,02 | | 14,91 |

Beispiel 35

5-Methyl-6-[2-(4-thiomorpholino)-benzoxazol-6-yl]-4,5-dihydro-3(2H)-pyridazinon

Hergestellt aus 5-Methyl-6-(2-methylthio-benzoxazol-6-yl)-4,5-dihydro-3(2H)-pyridazinon und Thiomorpholin analog Beispiel 11.
Ausbeute: 56,5% der Theorie,
Schmelzpunkt: 218°C

| | | | | | | | | |
|---|---|---|---|---|---|---|---|---|
| Ber.: | C | 58,16 | H | 5,49 | N | 16,96 | S | 9,70 |
| Gef.: | | 58,24 | | 5,49 | | 17,20 | | 9,54 |

Beispiel 36

5-Methyl-6-[2-(1-piperidino)-benzoxazol-6-yl]-4,5-dihydro-3(2H)-pyridazinon

Hergestellt aus 5-Methyl-6-(2-methylthio-benzoxazol-6-yl)-4,5-dihydro-3(2H)-pyridazinon und Piperidin analog Beispiel 11.
Ausbeute: 85,4% der Theorie,
Schmelzpunkt: 235°C

| | | | | | | |
|---|---|---|---|---|---|---|
| Ber.: | C | 65,37 | H | 6,45 | N | 17,94 |
| Gef.: | | 65,45 | | 6,50 | | 18,02 |

Beispiel 37

5-Methyl-6-[2-(1-oxido-4-thiomorpholino)-benzoxazol-6-yl]-4,5-dihydro-3(2H)-pyridazinon

Hergestellt aus 5-Methyl-6-(2-methylthio-benzoxazol-6-yl)-4,5-dihydro-3(2H)-pyridazinon und 1-Oxido-thiomorpholin analog Beispiel 11.
Ausbeute: 65,3% der Theorie,
Schmelzpunkt: 257–260°C

| | | | | | | | | | | |
|---|---|---|---|---|---|---|---|---|---|---|
| Ber.: | C | 55,48 | H | 5,24 | N | 16,17 | S | 9,26 | O | 13,86 |
| Gef.: | | 55,60 | | 5,20 | | 16,44 | | 9,44 | | 13,61 |

Beispiel 38

5-Methyl-6-[2-(1,1-dioxido-4-thiomorpholino)-benzoxazol-6-yl]-4, 5-dihydro-3(2H)-pyridazinon

Hergestellt aus 5-Methyl-6-(2-methylthio-benzoxazol-6-yl)-4,5-dihydro-3(2H)-pyridazinon und 1,1-Dioxido-thiomorpholin analog Beispiel 11.
Ausbeute: 63,5% der Theorie,
Schmelzpunkt: 280°C

| | | | | | | | | |
|---|---|---|---|---|---|---|---|---|
| Ber.: | C | 53,03 | H | 5,00 | N | 15,46 | S | 8,85 |
| Gef.: | | 53,00 | | 5,16 | | 15,51 | | 8,96 |

Beispiel 39

5-Methyl-6-[2-(4-methyl-1-piperazinyl)-benzoxazol-6-yl]-4,5-dihydro-3(2H)-pyridazinon

Hergestellt aus 5-Methyl-6-(2-methylthio-benzoxazol-6-yl)-4,5-dihydro-3(2H)-pyridazinon und N-Methyl-piperazin analog Beispiel 11.
Ausbeute: 61% der Theorie,
Schmelzpunkt: 198°C

| | | | | | | |
|---|---|---|---|---|---|---|
| Ber.: | C | 62,37 | H | 6,47 | N | 21,39 |
| Gef.: | | 62,62 | | 6,50 | | 21,44 |

Beispiel 40

5-Methyl-6-[2-(4-carbäthoxy-1-piperazinyl)-benzoxazol-6-yl]-4,5-dihydro-3(2H)-pyridazinon

Hergestellt aus 5-Methyl-6-(2-methylthio-benzoxazol-6-yl)-4,5-dihydro-3(2H)-pyridazinon und 4-Carbäthoxy-piperazin analog Beispiel 11.
Ausbeute: 72,7% der Theorie,
Schmelzpunkt: 239°C

| | | | | | | |
|---|---|---|---|---|---|---|
| Ber.: | C | 59,21 | H | 6,02 | N | 18,17 |
| Gef.: | | 59,53 | | 6,00 | | 18,37 |

Beispiel 41

5-Methyl-6-[2-(1-pyrrolidinyl)-benzoxazol-6-yl]-4,5-dihydro-3(2H)-pyridazinon

Hergestellt aus 5-Methyl-6-(2-methylthio-benzoxazol-6-yl)-4,5-dihydro-3(2H)-pyridazinon und Pyrrolidin analog Beispiel 11.
Ausbeute: 73,8% der Theorie,
Schmelzpunkt: 234°C

| | | | | | | |
|---|---|---|---|---|---|---|
| Ber.: | C | 64,41 | H | 6,08 | N | 18,78 |
| Gef.: | | 64,64 | | 6,07 | | 18,85 |

Beispiel 42

5-Methyl-6-[2-(1-piperazinyl)-benzoxazol-6-yl]-4,5-dihydro-3(2H)-pyridazinon

Hergestellt aus 5-Methyl-6-(2-methylthio-benzoxazol-6-yl)-4,5-dihydro-3(2H)-pyridazinon und Piperazin in der Schmelze analog Beispiel 11.
Ausbeute: 70,0% der Theorie,
Schmelzpunkt: 213°C

| | | | | | | |
|---|---|---|---|---|---|---|
| Ber.: | C | 61,33 | H | 6,11 | N | 22,35 |
| Gef.: | | 61,56 | | 6,01 | | 22,51 |

Beispiel 43

5-Methyl-6-[2-(4-methyl-1-imidazolyl)-benzoxazol-6-yl]-4,5-dihydro-3(2H)-pyridazinon

Hergestellt aus 5-Methyl-6-(2-methylthio-benzoxazol-6-yl)-4,5-dihydro-3(2H)-pyridazinon und 4-Methyl-imidazol bei 150°C analog Beispiel 11.
Ausbeute: 73,5% der Theorie,
Schmelzpunkt: 258°C

| | | | | | | |
|---|---|---|---|---|---|---|
| Ber.: | C | 62,13 | H | 4,89 | N | 22,64 |
| Gef.: | | 62,05 | | 4,86 | | 22,50 |

Beispiel 44

6-[2-(1-Piperidino)-benzoxazol-6-yl]-4,5-dihydro-3(2H)-pyridazinon

Hergestellt aus 6-(2-Methylthio-benzoxazol-6-yl)-4,5-dihydro-3(2H)-pyridazinon und Piperidin analog Beispiel 11.
Ausbeute: 62% der Theorie,
Schmelzpunkt: 198–199°C

| | | | | | | |
|---|---|---|---|---|---|---|
| Ber.: | C | 64,41 | H | 6,08 | N | 18,78 |
| Gef.: | | 64,62 | | 6,07 | | 18,61 |

Beispiel 45

6-[2-(4-Morpholino)-benzoxazol-6-yl]-4,5-dihydro-3(2H)-pyridazinon

Hergestellt aus 6-(2-Methylthio-benzoxazol-6-yl)-4,5-dihydro-3(2H)-pyridazinon und Morpholin analog Beispiel 11.
Ausbeute: 73,3% der Theorie,
Schmelzpunkt: 240°C

| | | | | | | |
|---|---|---|---|---|---|---|
| Ber.: | C | 59,99 | H | 5,37 | N | 18,66 |
| Gef.: | | 60,20 | | 5,37 | | 18,81 |

Beispiel 46

6-[2-(1-Pyrrolidinyl)-benzoxazol-6-yl]-4,5-dihydro-3(2H)-pyridazinon

Hergestellt aus 6-(2-Methylthio-benzoxazol-6-yl)-4,5-dihydro-3(2H)-pyridazinon und Pyrrolidin analog Beispiel 11.
Ausbeute: 78,9% der Theorie,
Schmelzpunkt: 265–266°C

| | | | | | | |
|---|---|---|---|---|---|---|
| Ber.: | C | 63,37 | H | 5,67 | N | 19,71 |
| Gef.: | | 63,50 | | 5,73 | | 19,55 |

Beispiel 47

6-[2-(4-Methyl-1-imidazolyl)-benzoxazol-6-yl]-4,5-dihydro-3(2H)-pyridazinon

Hergestellt aus 6-(2-Methylthio-benzoxazol-6-yl)-4,5-dihydro-3(2H)-pyridazinon und 4-Methyl-imidazol bei 150°C analog Beispiel 11.
Ausbeute: 17% der Theorie,
Schmelzpunkt: 280–282°C

| | | | | | | |
|---|---|---|---|---|---|---|
| Ber.: | C | 61,01 | H | 4,44 | N | 23,72 |
| Gef.: | | 60,80 | | 4,44 | | 23,49 |

Beispiel 48

6-[2-(4-Carbäthoxy-1-piperazinyl)-benzoxazol-6-yl]-4,5-dihydro-3(2H)-pyridazinon

Hergestellt aus 6-(2-Methylthio-benzoxazol-6-yl)-4,5-dihydro-3(2H)-pyridazinon und 4-Carbäthoxypiperazin analog Beispiel 11.
Ausbeute: 50,1% der Theorie,
Schmelzpunkt: 242–243°C

| | | | | | | |
|---|---|---|---|---|---|---|
| Ber.: | C | 58,21 | H | 5,70 | N | 18,86 |
| Gef.: | | 58,39 | | 5,84 | | 18,65 |

Beispiel 49

6-[2-(1-Piperazinyl)-benzoxazol-6-yl]-4,5-dihydro-3(2H)-pyridazinon

Hergestellt aus 6-(2-Methylthio-benzoxazol-6-yl)-4,5-dihydro-3(2H)-pyridazinon und Piperazin (Schmelze) analog Beispiel 11.
Ausbeute: 36,7% der Theorie,
Schmelzpunkt: 182–183°C

| | | | | | | |
|---|---|---|---|---|---|---|
| Ber.: | C | 60,19 | H | 5,72 | N | 23,40 |
| Gef.: | | 60,20 | | 5,60 | | 23,43 |

Beispiel 50

6-[2-(1-Oxido-4-thiomorpholino)-benzoxazol-6-yl]-4,5-dihydro-3(2H)-pyridazinon

Hergestellt aus 6-(2-Methylthio-benzoxazol-6-yl)-4,5-dihydro-3(2H)-pyridazinon und 1-Oxido-thiomorpholin analog Beispiel 11.
Ausbeute: 39,1% der Theorie,
Schmelzpunkt: > 310°C

| | | | | | | | | |
|---|---|---|---|---|---|---|---|---|
| Ber.: | C | 54,20 | H | 4,85 | N | 16,86 | S | 9,65 |
| Gef.: | | 54,35 | | 5,11 | | 16,87 | | 9,57 |

Beispiel 51

6-[2-(1,1-Dioxido-4-thiomorpholino)-benzoxazol-6-yl]-4,5-dihydro-3(2H)-pyridazinon

Hergestellt aus 6-(2-Methylthio-benzoxazol-6-yl)-4,5-dihydro-3(2H)-pyridazinon und 1,1-Dioxido-thiomorpholin analog Beispiel 11.
Ausbeute: 33,3% der Theorie,
Schmelzpunkt: 298–299°C

| | | | | | | | | |
|---|---|---|---|---|---|---|---|---|
| Ber.: | C | 51,71 | H | 4,63 | N | 16,08 | S | 9,20 |
| Gef.: | | 51,60 | | 4,83 | | 16,14 | | 9,30 |

Beispiel 52

6-[2-(4-Methyl-1-piperazinyl)-benzoxazol-6-yl]-4,5-dihydro-3(2H)-pyridazinon

Hergestellt aus 6-(2-Methylthio-benzoxazol-6-yl)-4,5-dihydro-3(2H)-pyridazinon und N-Methyl-piperazin analog Beispiel 11.
Ausbeute: 41,5% der Theorie,
Schmelzpunkt: 247–248°C

| | | | | | | |
|---|---|---|---|---|---|---|
| Ber.: | C | 61,33 | H | 6,11 | N | 22,35 |
| Gef.: | | 61,20 | | 6,31 | | 22,35 |

Beispiel 53

6-[2-(4-Thiomorpholino)-benzoxazol-6-yl]-4,5-dihydro-3(2H)-pyridazinon

Hergestellt aus 6-(2-Methylthio-benzoxazol-6-yl)-4,5-dihydro-3(2H)-pyridazinon und Thiomorpholin analog Beispiel 11.
Ausbeute: 39,2% der Theorie,
Schmelzpunkt: 253–254°C

| | | | | | | | | |
|---|---|---|---|---|---|---|---|---|
| Ber.: | C | 56,95 | H | 5,10 | N | 17,71 | S | 10,13 |
| Gef.: | | 56,87 | | 5,21 | | 17,68 | | 10,30 |

Beispiel 54

5-n-Propyl-6-[2-(1-imidazolyl)-benzoxazol-5-yl]-4,5-dihydro-3(2H)-pyridazinon

Hergestellt aus 5-n-Propyl-6-(2-methylthio-benzoxazol-5-yl)-4,5-dihydro-3(2H)-pyridazinon und Imidazol analog Beispiel 11.
Ausbeute: 30,3% der Theorie,
Schmelzpunkt: 190–192°C

| | | | | | | |
|---|---|---|---|---|---|---|
| Ber.: | C | 63,15 | H | 5,30 | N | 21,67 |
| Gef.: | | 63,09 | | 5,51 | | 21,52 |

Beispiel 55

5-n-Propyl-6-[2-(1-piperazinyl)-benzoxazol-5-yl]-4,5-dihydro-3(2H)-pyridazinon

Hergestellt aus 5-n-Propyl-6-(2-methylthio-benzoxazol-5-yl)-4,5-dihydro-3(2H)-pyridazinon und Piperazin analog Beispiel 11.
Ausbeute: 65,2% der Theorie,
Schmelzpunkt: 162–164°C

| | | | | | | |
|---|---|---|---|---|---|---|
| Ber.: | C | 63,33 | H | 6,79 | N | 20,52 |
| Gef.: | | 62,98 | | 6,93 | | 20,32 |

Beispiel 56

5-Methyl-6-[2-(1-imidazolyl)-benzoxazol-6-yl]-4,5-dihydro-3(2H)-pyridazinon

Hergestellt aus 5-Methyl-6-(2-methylthio-benzoxazol-6-yl)-4,5-dihydro-3(2H)-pyridazinon und Imidazol bei 160°C analog Beispiel 11.
Ausbeute: 17% der Theorie,
Schmelzpunkt: 219°C

| | | | | | | | | |
|---|---|---|---|---|---|---|---|---|
| Ber.: | C | 61,01 | H | 4,44 | N | 23,78 | O | 10,84 |
| Gef.: | | 61,00 | | 4,51 | | 23,63 | | 10,76 |

Beispiel 57

5-Methyl-6-[2-(4-morpholino)-benzoxazol-6-yl]-4,5-dihydro-3(2H)-pyridazinon

Hergestellt aus 5-Methyl-6-(2-methylthio-benzoxazol-6-yl)-4,5-dihydro-3(2H)-pyridazinon und Morpholin analog Beispiel 11.
Ausbeute: 81,2% der Theorie,
Schmelzpunkt: 209°C

| | | | | | | |
|---|---|---|---|---|---|---|
| Ber.: | C | 61,14 | H | 5,77 | N | 17,82 |
| Gef.: | | 60,93 | | 5,59 | | 17,91 |

Beispiel 58

6-[2-(3-Thienyl)-benzoxazol-6-yl]-4,5-dihydro-3(2H)-pyridazinon

Hergestellt aus N-[2-Hydroxy-4-(4,5-dihydro-3(2H)-pyridazinon-6-yl)-phenyl]-thiophen-3-carbonsäureamid und Eisessig/p-Toluolsulfonsäure analog Beispiel 23.
Ausbeute: 22,2% der Theorie,
Schmelzpunkt: 272–273°C

| | | | | | | | | |
|---|---|---|---|---|---|---|---|---|
| Ber.: | C | 60,59 | H | 3,73 | N | 14,13 | S | 10,78 |
| Gef.: | | 60,60 | | 3,68 | | 13,99 | | 10,66 |

Beispiel 59

5-Methyl-6-[2-(2-acetamido-5-pyridyl)-benzoxazol-6-yl]-4,5-dihydro-3(2H)-pyridazinon

Hergestellt aus N-[2-Hydroxy-4-(5-methyl-4,5-dihydro-3(2H)-pyridazinon-6-yl)-phenyl]-2-acetamino-pyridin-5-carbonsäureamid und Eisessig/p-Toluolsulfonsäure analog Beispiel 23.
Ausbeute: 12% der Theorie,
Schmelzpunkt: > 300°C

| | | | | | | |
|---|---|---|---|---|---|---|
| Ber.: | C | 62,80 | H | 4,72 | N | 19,27 |
| Gef.: | | 62,66 | | 4,96 | | 19,25 |

Beispiel 60

5-Methyl-6-[2-(2,6-dichlor-3-pyridyl)-benzoxazol-6-yl]-4,5-dihydro-3(2H)-pyridazinon

Hergestellt aus N-[2-Hydroxy-4-(5-methyl-4,5-dihydro-3(2H)-pyridazinon-6-yl)-phenyl]-2,6-dichlor-pyridin-3-carbonsäureamid und Eisessig/p-Toluolsulfonsäure analog Beispiel 23.
Ausbeute: 40% der Theorie,
Schmelzpunkt: 268–270°C

| | | | | | | | | |
|---|---|---|---|---|---|---|---|---|
| Ber.: | C | 54,42 | H | 3,22 | N | 14,93 | Cl | 18,90 |
| Gef.: | | 54,52 | | 3,33 | | 14,70 | | 18,88 |

Beispiel 61

5-n-Propyl-6-[2-(4-pyridyl)-benzoxazol-5-yl]-4,5-dihydro-3(2H)-pyridazinon

Hergestellt aus N-[2-Hydroxy-5-(5-n-propyl-4,5-dihydro-3(2H)-pyridazinon-6-yl)-phenyl]-pyridin-4-carbonsäureamid analog Beispiel 1.
Ausbeute: 40,1% der Theorie,
Schmelzpunkt: 200–202°C

| | | | | | | |
|---|---|---|---|---|---|---|
| Ber.: | C | 68,24 | H | 5,42 | N | 16,76 |
| Gef.: | | 68,26 | | 5,38 | | 16,91 |

Beispiel 62

5-Methyl-6-[2-(2-chlor-6-morpholino-3-pyridyl)-benzoxazol-6-yl]-4,5-dihydro-3(2H)-pyridazinon

Hergestellt aus 5-Methyl-6-[2,6-dichlor-3-pyridyl)-benzoxazol-6-yl]-4,5-dihydro-3(2H)-pyridazinon und Morpholin analog Beispiel 21.
Ausbeute: 22% der Theorie,
Schmelzpunkt: 236°C

| | | | | | | | | |
|---|---|---|---|---|---|---|---|---|
| Ber.: | C | 59,22 | H | 4,73 | N | 16,45 | Cl | 8,39 |
| Gef.: | | 59,26 | | 4,82 | | 16,63 | | 8,58 |

Beispiel 63

5-Methyl-6-[2-(1-imidazolyl)-benzimidazol-5-yl]-4,5-dihydro-3(2H)-pyridazinon

a) 4-(2-Chlor-benzimidazol-5-yl)-4-oxo-3-methyl-buttersäuremethylester

12 g (46 mMol) 4-(Benzimidazol-2-on-5-yl)-4-oxo-3-methyl-buttersäuremethylester werden in 200 ml Phosphoroxychlorid 1,5 Stunden lang unter Rückfluß erhitzt. Anschließend wird das überschüssige Phosphoroxychlorid im Vakuum abdestilliert und der verbleibende Rückstand unter Eiskühlung mit Wasser zersetzt. Man neutralisiert mit Ammoniak, extrahiert mit Essigester, wäscht die Essigesterphase

zweimal mit Wasser, trocknet sie über Magnesiumsulfat und engt zur Trockne ein. Der Rückstand wird durch Chromatographie gereinigt (Kieselgel: 0, 032-0, 063 mm, Elutionsmittel: Essigester/Petroläther = 2/1).

Ausbeute: 7,9 g (61,2% der Theorie),
Schmelzpunkt: 135–136°C

b) 4-[2-(1-Imidazolyl)-benzimidazol-5-yl]-4-oxo-3-methyl-buttersäuremethylester

1,5 g (5,3 mMol) 4-(2-Chlor-benzimidazol-5-yl)-4-oxo-3-methyl-buttersäuremethylester und 1,1 g (16 mMol) Imidazol werden vermischt und 1,5 Stunden lang auf 130°C erhitzt. Man erhält eine klare zähe Schmelze, die nach Abkühlen mit Wasser versetzt wird. Man extrahiert dreimal mit Essigester, wäscht die vereinigten Essigesterlösungen dreimal mit Wasser, trocknet sie über Magnesiumsulfat und dampft zur Trockne ein.

Ausbeute: 1,5 g (90% der Theorie),
Schmelzpunkt: 130–140°C

| | | | | | | |
|---|---|---|---|---|---|---|
| Ber.: | C | 61,53 | H | 5,16 | N | 17,94 |
| Gef.: | | 61,21 | | 5,47 | | 17,87 |

c) 5-Methyl-6-[2-(1-imidazolyl)-benzimidazol-5-yl]-4,5-dihydro-3(2H)-pyridazinon

1,4 g (4,3 mMol) 4-[2-(1-Imidazolyl)-benzimidazol-5-yl]-4-oxo-3-methyl-buttersäuremethylester werden mit 30 ml Essigsäure und 3,8 ml 80%igem Hydrazinhydrat 1,25 Stunden lang auf 105°C erhitzt. Nach Abkühlen wird mit ca. 100 ml Wasser versetzt und dreimal mit Essigester extrahiert. Die vereinigten Essigesterlösungen werden zweimal mit Wasser gewaschen, über Magnesiumsulfat getrocknet und eingedampft. Der Rückstand wird chromatographisch gereinigt (Kieselgel: 0,032–0,063 mm, Elutionsmittel: Methylenchlorid/Ethanol = 1:0 bis 1:0,14).

Ausbeute: 0,29 g (22,9% der Theorie),
Schmelzpunkt: 282–285°C (Zers.)

Beispiel 64

5-Methyl-6-[2-(4-methyl-1-piperazinyl)-benzimidazol-5-yl]-4,5-dihydro-3(2H)-pyridazinon

Hergestellt aus 4-[2-(4-Methyl-1-piperazinyl)-benzimidazol-5-yl]-4-oxo-3-methyl-buttersäuremethylester und Hydrazinhydrat analog Beispiel 63c.

Ausbeute: 67% der Theorie,
Schmelzpunkt: 317–320°C (Zers.)

Beispiel 65

5-Methyl-6-[2-(4-morpholino)-benzimidazol-5-yl]-4,5-dihydro-3(2H)-pyridazinon

Hergestellt aus 4-[2-(4-Morpholino)-benzimidazol-5-yl]-4-oxo-3-methyl-buttersäuremethylester und Hydrazinhydrat analog Beispiel 63c.

Ausbeute: 51% der Theorie,
Massenspektrum: $M^+$ = 313 m/e
IR-Spektrum (Methylenchlorid): 1665 $cm^{-1}$ (Carbonyl)

Beispiel 66

5-Methyl-6-[2-(1-pyrrolidinyl)-benzimidazol-5-yl]-4,5-dihydro-3(2H)-pyridazinon

Hergestellt aus 4-[2-(1-Pyrrolidinyl)-benzimidazol-5-yl]-4-oxo-3-methyl-buttersäuremethylester und Hydrazinhydrat analog Beispiel 63c.

Ausbeute: 75,3% der Theorie,
Schmelzpunkt: 233–236°C

| | | | | | | |
|---|---|---|---|---|---|---|
| Ber.: | C | 64,63 | H | 6,44 | N | 23,55 |
| Gef.: | | 64,42 | | 6,67 | | 23,36 |

Beispiel 67

5-Methyl-6-[2-(4-thiomorpholino)-benzimidazol-5-yl]-4,5-dihydro-3(2H)-pyridazinon

Hergestellt aus 4-[2-(4-Thiomorpholino)-benzimidazol-5-yl]-4-oxo-3-methyl-buttersäuremethylester und Hydrazinhydrat analog Beispiel 63c.
Ausbeute: 73% der Theorie,

| | |
|---|---|
| UV-Spektrum (Ethanol): | 224 nm (0,18), 246 nm (0,28), 323 nm (0,33), |
| IR-Spektrum (Methylenchlorid): | 1665 $cm^{-1}$ (Carbonyl) 1625–1630 $cm^{-1}$ (C=N) |

Beispiel 68

5-Methyl-6-[2-(1-oxido-4-thiomorpholino)-benzimidazol-5-yl]-4,5-dihydro-3(2H)-pyridazinon

Hergestellt aus 4-[2-(1-Oxido-4-thiomorpholino)-benzimidazol-5-yl]-4-oxo-3-methyl-buttersäuremethylester und Hydrazinhydrat analog Beispiel 63c.
Ausbeute: 84% der Theorie,
Schmelzpunkt: 105°C (Zers.)

| | |
|---|---|
| UV-Spektrum (Ethanol): | 221 nm (0,34), 245 nm (0,53), 319 nm (0,63), |
| IR-Spektrum (Methylenchlorid): | 1655 $cm^{-1}$ (Carbonyl) |

Beispiel 69

5-Methyl-6-[2-(1-hexahydroazepinyl)-benzimidazol-5-yl]-4,5-dihydro-3(2H)-pyridazinon

Hergestellt aus 4-[2-(1-Hexahydroazepinyl)-benzimidazol-5-yl]-4-oxo-3-methyl-buttersäuremethylester und Hydrazinhydrat analog Beispiel 63c.
Ausbeute: 19% der Theorie,
Schmelzpunkt: 295–298°C (Zers.)
Massenspektrum: $M^+$: 325 m/e

Beispiel 70

5-Methyl-6-[2-(1-pyrazolyl)-benzimidazol-5-yl]-4,5-dihydro-3(2H)-pyridazinon

Hergestellt aus 4-[2-(1-Pyrazolyl)-benzimidazol-5-yl]-4-oxo-3-methyl-buttersäuremethylester und Hydrazinhydrat analog Beispiel 63c.
Ausbeute: 37% der Theorie,
Schmelzpunkt: 290–292°C

| | | | | | | |
|---|---|---|---|---|---|---|
| Ber.: | C | 61,21 | H | 4,80 | N | 28,56 |
| Gef.: | | 61,17 | | 4,89 | | 28,49 |

Beispiel 71

5-Methyl-6-[2-(4-acetyl-1-piperazinyl)-benzimidazol-5-yl]-4,5-dihydro-3(2H)-pyridazinon

Hergestellt aus 4-[2-(4-Acetyl-1-piperazinyl)-benzimidazol-5-yl]-4-oxo-3-methyl-buttersäuremethylester und Hydrazinhydrat analog Beispiel 63c.
Ausbeute: 44% der Theorie,
Schmelzpunkt: 295–302°C

| | | | | | | |
|---|---|---|---|---|---|---|
| Ber.: | C | 61,00 | H | 6,26 | N | 23,71 |
| Gef.: | | 61,30 | | 6,43 | | 23,56 |

Beispiel 72

5-Methyl-6-[2-(imidazolidin-2-on-1-yl)-benzimidazol-5-yl]-4,5-dihydro-3(2H)-pyridazinon

Hergestellt aus 4-[2-(Imidazolidin-2-on-1-yl)-benzimidazol-5-yl]-4-oxo-3-methyl-buttersäuremethylester und Hydrazinhydrat analog Beispiel 63c.
Ausbeute: 7% der Theorie,
Schmelzpunkt: 285°C (Zers.)
Massenspektrum: $M^+$ = 312 m/e

Beispiel 73

5-Methyl-6-[2-(1-piperidino)-benzimidazol-5-yl]-4,5-dihydro-3(2H)-pyridazinon

Hergestellt aus 4-[2-(1-piperidino)-benzimidazol-5-yl]-4-oxo-3-methyl-buttersäuremethylester und Hydrazinhydrat analog Beispiel 63c.
Ausbeute: 49% der Theorie,
Schmelzpunkt: 185°C (Zers.)
Massenspektrum: $M^+$ = 311 m/e

Beispiel 74

5-Methyl-6-[2-(3-hydroxy-4-methyl-2-pyrazolyl)-benzimidazol-5-yl]-4,5-dihydro-3(2H)-pyridazinon

Hergestellt aus 4-[2-(3-Hydroxy-4-methyl-2-pyrazolyl)-benzimidazol-5-yl]-4-oxo-3-methyl-buttersäuremethylester und Hydrazinhydrat analog Beispiel 63c.
Ausbeute: 67% der Theorie,
Schmelzpunkt: 260–265°C (Zers.)
Massenspektrum: $M^+$ = 324 m/e

Beispiel 75

5-Methyl-6-[2-(4-morpholino)-benzimidazol-5-yl]-4,5-dihydro-3(2H)-pyridazinon

a) 4-[2-(4-Morpholino)-benzimidazol-5-yl]-4-oxo-3-methylbuttersäuremorpholid

Hergestellt aus 4-(2-Sulfo-benzimidazol-5-yl)-4-oxo-3-methylbuttersäure analog Beispiel 63a.
Ausbeute: 30% der Theorie,
Massenspektrum: $M^+$ = 386 m/e

| | | | | | | | |
|---|---|---|---|---|---|---|---|
| Ber.: | C | 62,16 | H | 6,78 | N | 14,50 |
| Gef.: | | 61,90 | | 6,86 | | 14,36 |

b) 5-Methyl-6-[2-(4-morpholino)-benzimidazol-5-yl]-4,5-dihydro-3(2H)-pyridazinon

Hergestellt aus 4-[2-(4-Morpholino)-benzimidazol-5-yl]-4-oxo-3-methyl-buttersäuremorpholid und Hydrazinhydrat analog Beispiel 63c.
Ausbeute: 61% der Theorie,
Massenspektrum: $M^+$ = 313 m/e

Beispiel 76

5-Methyl-6-[2-(1[1.2.4]triazolyl)-benzimidazol-5-yl]-4,5-dihydro-3(2H)-pyridazinon

a) 4-[1-[1.2.4]Triazolyl-benzimidazol-5-yl]-4-oxo-3-methyl-buttersäuremethylester

260 mg (1 mMol) 4-(2-Chlor-benzimidazol-5-yl)-4-oxo-3-methyl-buttersäuremethylester werden mit 980 mg (14 mMol) 1,2,4-Triazol im Ölbad auf 160°C erhitzt. Nach 45 Minuten wird abgekühlt und mit 50 ml heißem Methanol verrieben. Anschließend destilliert man 10 bis 20 ml Methanol ab, saugt den erhaltenen Niederschlag noch warm ab und trocknet ihn an der Luft.
Ausbeute: 0,22 g (74% der Theorie),
Schmelzpunkt: 300-306°C (Zers.)

b) 5-Methyl-6-[2-(1-[1.2.4]triazolyl)-benzimidazol-5-yl]-4,5-dihydro-3(2H)-pyridazinon

280 mg (1 mMol) 4-[1-[1.2.4]Triazolyl-benzimidazol-5-yl]-4-oxo-3-methyl-buttersäuremethylester werden mit 5 ml Äthanol und 1 ml 98%igem Hydrazinhydrat eine Stunde lang auf 100°C erhitzt. Anschließend destilliert man das Lösungsmittel im Vakuum ab, versetzt den Rückstand mit Wasser und extrahiert 3mal mit Essigester. Die vereinigten Essigesterphasen werden 2mal mit Wasser gewaschen, über Natriumsulfat getrocknet und im Vakuum eingeengt. Der erhaltene Rückstand wird chromatographisch gereinigt (Kieselgel, Elutionsmittel: Essigester).
Ausbeute: 107 mg (40% der Theorie),
Schmelzpunkt: 228–230°C.

Beispiel 77

5-Methyl-6-[2-(3-methyl-1-pyrazolyl)-benzimidazol-5-yl]-4,5-dihydro-3(2H)-pyridazinon

Hergestellt aus 5-Methyl-6-(2-chlor-benzimidazol-5-yl)-4,5-dihydro-3(2H)-pyridazinon und 3-Methyl-pyrazol analog Beispiel 76b.
Ausbeute: 87% der Theorie,
Schmelzpunkt: 320–322°C (Zers.)

| | | | | | | |
|---|---|---|---|---|---|---|
| Ber.: | C | 62,32 | H | 5,23 | N | 27,26 |
| Gef.: | | 62,32 | | 5,21 | | 27,43 |

Beispiel 78

5-Methyl-6-[2-(1-pyrrolidinyl)-benzimidazol-5-yl]-4,5-dihydro-3-(2H)-pyridazinon

295 mg (1 mMol) 5-Methyl-6-[2-(1-pyrrolidinyl)-benzimidazol-5-yl]-3(2H)-pyridazinon werden in 20 ml Eisessig gelöst und bei Raumtemperatur und 5 bar Wasserstoff in Gegenwart von Platindioxid hydriert. Nach Beendigung der Wasserstoffaufnahme wird vom Katalysator abfiltriert und das Filtrat im Vakuum zur Trockne eingeengt. Der Rückstand wird durch Chromatographie gereinigt (Kieselgel, Elutionsmittel:Methylenchlorid/Ethanol = 1:0 bis 1:0,2).
Ausbeute: 30% der Theorie,
Schmelzpunkt: 233–236°C

Beispiel I

Dragées mit 5 mg 5-Methyl-6-[2-(1-imidazolyl)-benzoxazol-5-yl]-4,5-dihydro-3(2H)-pyridazinon

1 Dragéekern enthält:

| | |
|---|---|
| Wirksubstanz | 5,0 mg |
| Milchzucker | 25,2 mg |
| Maisstärke | 18,0 mg |
| Cellulose, mikrokristallin | 10,0 mg |
| Polyvinylpyrrolidon | 1,0 mg |
| Magnesiumstearat | 0,8 mg |
| | 60,0 mg |

Herstellverfahren:

Der Wirkstoff, Milchzucker, Maisstärke, Cellulose und Polyvinylpyrrolidon werden gemischt und mit Wasser granuliert. Das Granulat wird bei einer Temperatur von 45° C getrocknet und die angegebene Menge Magnesiumstearat zugemischt. Auf einer Tablettiermaschine werden bikonvexe Kerne mit einem Durchmesser von 5 mm hergestellt.

Dragierung:

Die Kerne werden mit einer 15%igen Polyvinylpyrrolidon-Lösung überzogen und mit Dragiersuspension bis zu einem Gewicht von 80 mg fertigdragiert.

Beispiel II

Tabletten mit Teilkerbe zu 50 mg 5-Methyl-6-[2-(imidazol-1-yl)-benzoxazol-5-yl]-4,5-dihydro-3(2H)-pyridazinon

Zusammensetzung:
1 Tablette enthält:

| | |
|---|---|
| Wirksubstanz | 50,0 mg |
| Milchzucker | 40,0 mg |
| Maisstärke | 17,0 mg |
| Polyvinylpyrrolidon | 2,0 mg |
| Magnesiumstearat | 1,0 mg |
| | 110,0 mg |

Herstellungsverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50° C wird erneut gesiebt (1,5 mm-Maschenweite) und das Schmiermittel zugemischt.

Die preßfertige Mischung wird zu Tabletten mit Teilkerbe verarbeitet.

Beispiel III

Suppositorien mit 100 mg 5-Methyl-6-[2-(1-imidazolyl)-benzoxazol-5-yl]-4,5-dihydro-3(2H)-pyridazinon

1 Zäpfchen enthält:

| | |
|---|---|
| Wirksubstanz | 100,0 mg |
| Suppositorienmassen (Hartfett) | 1600,0 mg |
| | 1700,0 mg |

Herstellungsverfahren:

Die feinpulverisierte Wirksubstanz wird in die geschmolzene und auf 40°C abgekühlte Zäpfchenmasse einhomogenisiert. Man kühlt auf 37°C ab und gießt die Masse in leicht vorgekühlte Formen aus.

Zäpfchengewicht: 1,7 g.

Beispiel IV

Trockenampullen zu 10 mg 5-Methyl-6-[2-(1-imidazolyl)-benzoxazol-5-yl]-4,5-dihydro-3(2H)-pyridazinon

Zusammensetzung:

a) Trockenampullen:

| | |
|---|---|
| Wirksubstanz | 10,0 mg |
| Aqua bidest. | ad 1,0 ml |

Herstellung:

Die Substanz wird in Wasser gelöst, unter aseptischen Bedingungen durch einen Membranfilter filtriert und anschließend in braune, gereinigte und sterilisierte 2 ml-Injektionsfläschchen abgefüllt.

In einer geeigneten, aseptisch vorbehandelten Gefriertrockenanlage werden die Lösungen eingefroren und lyophilisiert sowie (durch Hydraulik) maschinell verschlossen, danach verbördelt.

b) Lösungsampullen:

| | |
|---|---|
| Zitronensäure × 1 $H_2O$ | 1,0 mg |
| $Na_2HPO_4$ × 12$H_2O$ | 20,0 mg |
| NaCl | 6,0 mg |
| Aqua bidest. | ad 1,0 ml |

Herstellung:

Die Substanzen werden der Reihe nach gelöst. Nach Filtration durch einen Membranfilter wird die Lösung in braune, gereinigte und sterilisierte 1 ml-Ampullen abgefüllt. Die Ampullen werden bei 120°C 20 Minuten autoklaviert. Anschließende Kontrolle auf Dichtigkeit und Faserfreiheit.

**Patentansprüche**

1. Pyridazinone der allgemeinen Formel

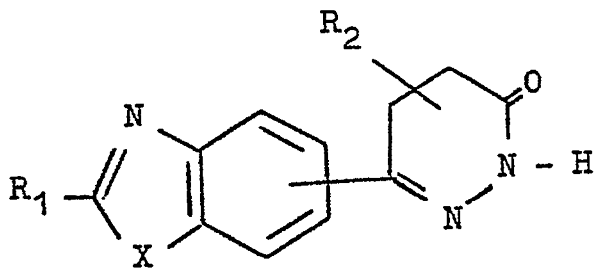

,(I)

in der
X ein Sauerstoff- oder Schwefelatom,
$R_1$ einen über ein Stickstoffatom gebundenen gesättigten 5- bis 7-gliedrigen Alkyleniminoring oder einen über ein Kohlenstoffatom gebundenen 5- bis 7-gliedrigen gesättigten Imino-, N-Alkyl-imino-, N-Phenylalkyl-imino-, N-Alkanoylimino- oder N-Alkoxycarbonyl-imino-alkylenring, wobei die vorstehend erwähnten Ringe im Kohlenstoffgerüst durch ein oder zwei Alkylgruppen substituiert und eine Methylengruppe in 4-Stellung eines 6- oder 7-gliedrigen Ringes durch ein Sauerstoff- oder Schwefelatom, eine Sulfinyl-, Sulfonyl-, Imino-, Alkylimino-, Phenylalkylimino-, Alkoxycarbonylimino- oder Alkanoyliminogruppe ersetzt sein kann, einen gegebenenfalls durch eine oder zwei Alkylgruppen über ein Stickstoffatom gebundenen gesättigten 5- bis 7-gliedrigen Alkyleniminoring, in dem eine $-CH_2CH_2-$Gruppe durch eine –NH–CO–Gruppe ersetzt ist, wobei die CO–Gruppe dieser –NH–CO–Gruppe mit dem bereits vorhandenen N-Atom verknüpft ist, einen über ein Stickstoffatom gebundenen 5-gliedrigen heteroaromatischen Ring, welcher zusätzlich ein Sauerstoff- oder Schwefelatom, ein oder zwei Stickstoffatome, ein Sauerstoff- oder Schwefelatom und ein Stickstoffatom enthalten kann, wobei der heteroaromatische Ring durch eine oder zwei Alkylgruppen und im Falle der Pyrazole auch durch eine Hydroxygruppe und eine Alkylgruppe substituiert sein kann, einen über ein Kohlenstoffatom gebundenen 5- oder 6-gliedrigen heteroaromatischen Ring, welcher ein Sauerstoff- oder Schwefelatom, ein, zwei oder drei Stickstoffatome, ein Sauerstoff- oder Schwefelatom und ein oder zwei Stickstoffatome enthalten kann, wobei die heteroaromatischen Ringe durch ein oder zwei Alkylgruppen, durch eine Amino- oder Alkanoylaminogruppe und im Falle der Pyridine auch durch ein oder zwei Halogenatome oder durch ein Halogenatom und eine Amino- oder Morpholinogruppe substituiert sein können, und
$R_2$ ein Wasserstoffatom oder eine Alkylgruppe oder
X eine gegebenenfalls durch eine Alkyl- oder Phenylalkylgruppe substituierte Iminogruppe,
$R_1$ einen über ein Stickstoffatom gebundenen gesättigten 5- bis 7-gliedrigen Alkyleniminoring oder einen über ein Kohlenstoffatom gebundenen 5- bis 7-gliedrigen gesättigten Imino-, N-Alkyl-imino-, N-Phenylalkyl-imino-, N-Alkanoylimino- oder N-Alkoxycarbonyl-imino-alkylenring, wobei die vorstehend erwähnten Ringe im Kohlenstoffgerüst durch eine oder zwei Alkylgruppen substituiert und eine Methylengruppe in 4-Stellung eines 6- oder 7-gliedrigen Ringes durch ein Sauerstoff- oder Schwefelatom, eine Sulfinyl-, Sulfonyl-, Imino-, Alkylimino-, Phenylalkylimino-, Alkoxycarbonylimino- oder Alkanoyliminogruppe ersetzt sein kann, einen gegebenenfalls durch eine oder zwei Alkylgruppen über ein Stickstoffatom gebundenen gesättigten 5- bis 7-gliedrigen Alkyleniminoring, in dem eine $-CH_2CH_2-$Gruppe durch eine –NH–CO–Gruppe ersetzt ist, wobei die CO–Gruppe dieser –NH–CO–Gruppe mit dem bereits vorhandenen N-Atom verknüpft ist, und
$R_2$ ein Wasserstoffatom oder eine Alkylgruppe, wobei alle vorstehend erwähnten Alkyl-, Alkanoyl- oder Alkoxygruppen jeweils 1 bis 3 Kohlenstoffatome enthalten können, oder
X eine Iminogruppe,

$R_1$ eine 1-Imidazolyl-, 1-Pyrazolyl-, 3-Methyl-1-pyrazolyl-, 3-Hydroxy-4-methyl-2-pyrazolyl- oder 1-[1.2.4]Triazolylgruppe und
$R_2$ eine Methylgruppe in 5-Stellung oder
X eine Iminogruppe,
$R_1$ eine 2-Chlor-6-morpholino-4-pyridylgruppe und
$R_2$ ein Wasserstoffatom bedeuten,
deren optisch aktive Antipoden, wenn $R_2$ kein Wasserstoffatom darstellt, und deren Säureadditionssalze.

2. Pyridazinone der allgemeinen Formel I gemäß Anspruch 1, in der
X ein Sauerstoff- oder Schwefelatom,
$R_1$ eine über ein Stickstoffatom gebundene Pyrrolidinyl-, Piperidino-, 1-Hexahydroazepinyl-, Morpholino-, Thiomorpholino-, 1-Oxidothiomorpholino-, 1,1-Dioxidothiomorpholino-, 1-Piperazinyl-, 4-Methyl-1-piperazinyl-, 4-Acetyl-1-piperazinyl-, 4-Carbäthoxy-1-piperazinyl-, 1-Imidazolyl-, 1-Pyrazolyl-, 1-[1.2.4]Triazolyl-, Imidazolidin-2-on-1-yl- oder 3-Hydroxy-2-pyrazolylgruppe oder eine über ein Kohlenstoffatom gebundene Piperidino-, N-Acetyl-piperidino-, Furyl-, Thienyl-, Pyrrolyl-, Pyridyl-, Amino-pyridyl-, Acetylaminopyridyl-, Pyrimidyl-, Pyrazinyl-, Oxazolyl-, Dihalogen-pyridyl- oder Halogen-morpholino-pyridylgruppe, wobei alle vorstehend erwähnten Reste im Kohlenstoffgerüst durch eine Methylgruppe substituiert sein können, und
$R_2$ ein Wasserstoffatom oder eine Methylgruppe in 5-Stellung oder
X die Iminogruppe,
$R_1$ eine über ein Stickstoffatom gebundene Pyrrolidinyl-, Piperidino-, 1-Hexahydroazepinyl-, Morpholino-, Thiomorpholino-, 1-Oxidothiomorpholino-, 1,1-Dioxidothiomorpholino-, 1-Piperazinyl-, 4-Methyl-1-piperazinyl-, 4-Acetyl-1-piperazinyl-, 4-Carbäthoxy-1-piperazinyl- oder Imidazolidin-2-on-1-yl-gruppe oder eine über ein Kohlenstoffatom gebundene Piperidino- oder N-Acetyl-piperidinogruppe, wobei alle vorstehend erwähnten Reste im Kohlenstoffgerüst durch eine Methylgruppe substituiert sein können, und
$R_2$ ein Wasserstoffatom oder eine Methylgruppe in 5-Stellung oder
X eine Iminogruppe,
$R_1$ eine 1-Imidazolyl-, 1-Pyrazolyl-, 3-Methyl-1-pyrazolyl-, 3-Hydroxy-4-methyl-2-pyrazolyl- oder 1-[1.2.4]Triazolylgruppe und
$R_2$ eine Methylgruppe in 5-Stellung oder
X eine Iminogruppe,
$R_1$ eine 2-Chlor-6-morpholino-4-pyridylgruppe und
$R_2$ ein Wasserstoffatom bedeuten,
deren optisch aktive Antipoden, wenn $R_2$ kein Wasserstoffatom darstellt, und deren Säureadditionssalze.

3. Pyridazinone der allgemeinen Formel I gemäß Anspruch 1, in der
X ein Sauerstoffatom,
$R_1$ die 1-Pyrrolidinyl-, 1-Imidazolyl-, 2-Furyl-, 3-Thienyl-, 1-Piperazinyl-, 4-Methyl-1-piperazinyl-, 4-Carbäthoxy-1-piperazinyl-, 4-Morpholino-, 4-Thiomorpholino-, 1-Oxido-4-thiomorpholino- oder 4-Pyridylgruppe und
$R_2$ in 5-Stellung die Methylgruppe oder
X die Iminogruppe,
$R_1$ die 1-Pyrrolidinyl-, 1-Piperazinyl-, 4-Methyl-1-piperazinyl-, 4-Carbäthoxy-1-piperazinyl-, 4-Morpholino-, 4-Thiomorpholino- oder 1-Oxido-4-thiomorpholinogruppe und
$R_2$ in 5-Stellung die Methylgruppe bedeuten, deren optisch aktiven Antipoden, wenn $R_2$ kein Wasserstoffatom darstellt, und deren Säureadditionssalze.

4. 5-Methyl-6-[2-(1-imidazolyl)-benzoxazol-5-yl]-4,5-dihydro-3(2H)-pyridazinon, dessen optisch aktive Antipoden und dessen Säureadditionssalze.

5. 5-Methyl-6-[2-(1-piperazinyl)-benzoxazol-6-yl]-4,5-dihydro-3(2H)-pyridazinon, dessen optisch aktive Antipoden und dessen Säureadditionssalze.

6. Physiologisch verträgliche Säureadditionssalze der Verbindungen gemäß den Ansprüchen 1 bis 5 mit anorganischen oder organischen Säuren.

7. Arzneimittel, enthaltend eine Verbindung der allgemeinen Formel I gemäß den Ansprüchen 1 bis 5 oder dessen physiologisch verträgliches Säureadditionssalz gemäß Anspruch 6 neben einem oder mehreren inerten Trägerstoffen und/oder Verdünnungsmitteln.

8. Verfahren zur Herstellung eines Arzneimittels gemäß Anspruch 7, dadurch gekennzeichnet, daß auf nichtchemischem Wege eine Verbindung gemäß den Ansprüchen 1 bis 5 oder dessen physiologisch verträgliches Säureadditionssalz gemäß Anspruch 6 in einen oder mehrere inerte Trägerstoffe und/oder Verdünnungsmittel eingearbeitet wird.

9. Verwendung einer Verbindung gemäß den Ansprüchen 1 bis 6 zur Herstellung eines Arzneimittels, welches zur Behandlung der chronischen Herzinsuffizienz, der Angina Pectoris und zur Prophylaxe arterieller Thromboembolien und arterieller Verschlußkrankheiten geeignet ist.

10. Verfahren zur Herstellung der Verbindungen gemäß den Ansprüchen 1 bis 6, dadurch gekennzeichnet, daß

a) zur Herstellung von Verbindungen der allgemeinen Formel I, in der $R_1$ einen der eingangs erwähnten über ein Kohlenstoffatom gebundenen Reste darstellt, eine gegebenenfalls im Reaktionsgemisch gebildete Verbindung der allgemeinen Formel

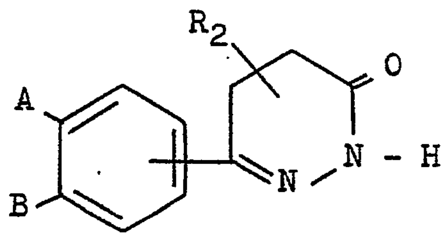

,(II)

in der $R_2$ wie in den Ansprüchen 1 bis 3 definiert ist,
einer der Reste A oder B eine

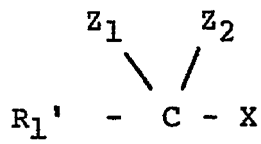

- Gruppe

und
der andere der Reste A oder B eine Aminogruppe oder eine

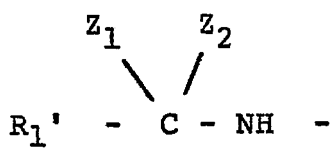

oder

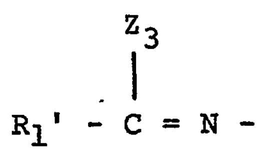

- Gruppe

bedeuten, wobei X wie in den Ansprüchen 1 bis 3 definiert ist und
$R_1$' einen der für $R_1$ in den Ansprüchen 1 bis 3 erwähnten über einen Kohlenstoffatom gebundenen Reste darstellt
$Z_1$ und $Z_2$ die gleich oder verschieden sein können, nukleophile Austrittsgruppen oder
$Z_1$ oder $Z_2$ zusammen ein Sauerstoff- oder Schwefelatom, eine gegebenenfalls durch eine Alkyl- oder Phenylalkylgruppe substituierte Iminogruppe, wobei die vorstehend erwähnten Alkylteile jeweils 1 bis 3 Kohlenstoffatome enthalten können, oder eine Alkylendioxigruppe mit 2 oder 3 Kohlenstoffatomen und
$Z_3$ die für $Z_1$ oben erwähnten Bedeutungen besitzt oder eine Imidazolylgruppe, ein Chlor-, Brom- oder Jodatom bedeuten, cyclisiert wird oder
b) eine Carbonsäure der allgemeinen Formel

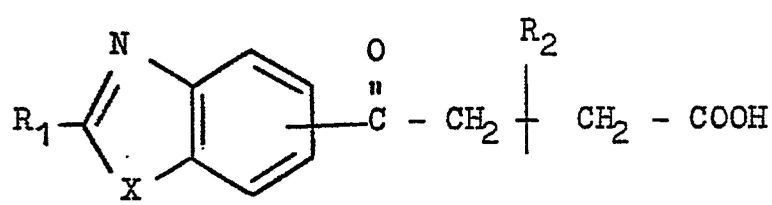

,(III)

in der
$R_1$ und $R_2$ wie in den Ansprüchen 1 bis 3 definiert sind, oder deren reaktionsfähige Säurederivate mit Hydrazin umgesetzt wird oder
c) zur Herstellung von Verbindungen der allgemeinen Formel I, in der $R_1$ einen der in den Ansprüchen 1 bis 3 erwähnten, über ein Stickstoffatom gebundenen Reste darstellt, eine Verbindung der allgemeinen Formel

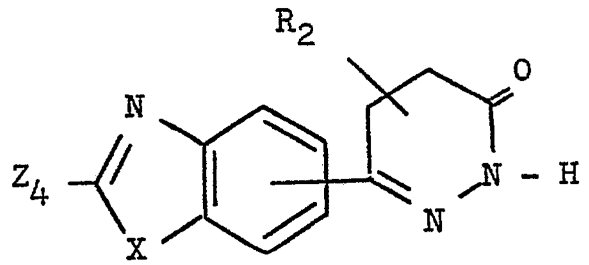

,(IV)

in der

$R_2$ und X wie in den Ansprüchen 1 bis 3 definiert sind und $Z_4$ eine nukleophil austauschbare Gruppe bedeutet, mit einem Amin der allgemeinen Formel

$$H{-}R_1'', \qquad (V)$$

in der

$R_1''$ einen der für $R_1$ in den Ansprüchen 1 bis 3 erwähnten über ein Stickstoffatom gebundenen Rest darstellt, umgesetzt wird oder

d) zur Herstellung von Verbindungen der allgemeinen Formel I, in der X ein Sauerstoffatom oder eine gegebenenfalls durch eine Alkyl- oder Phenylalkylgruppe substituierte Iminogruppe darstellt, eine Verbindung der allgemeinen Formel

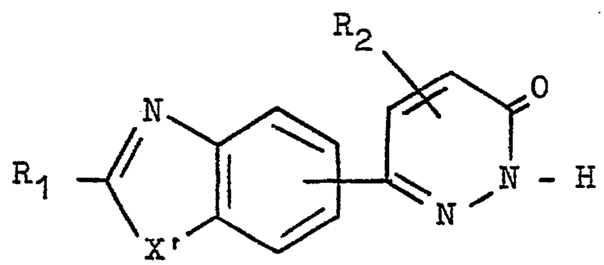

,(VI)

in der

$R_1$ und $R_2$ wie in den Ansprüchen 1 bis 3 definiert sind und X, ein Sauerstoffatom oder eine gegebenenfalls durch eine Alkyl- oder Phenylalkylgruppe substituierte Iminogruppe, wobei der Alkylteil jeweils 1 bis 5 Kohlenstoffatome enthalten kann, bedeutet, hydriert wird und

gewünschtenfalls anschließend eine so erhaltene Verbindung der allgemeinen Formel I, in der $R_1$ eine Imino- und/oder Aminogruppe enthält, mittels Alkanoylierung in eine entsprechende Verbindung der allgemeinen Formel I, in der $R_1$ eine Alkanoylimino- und/oder Alkanoylaminogruppe enthält, übergeführt wird oder

eine so erhaltene Verbindung der allgemeinen Formel I, in der $R_1$ einen über ein Kohlenstoffatom gebundenen, durch Halogenatome substituierten Pyridinrest darstellt, mittels katalytischer Enthalogenierung und Hydrierung in eine Verbindung der allgemeinen Formel I, in der $R_1$ einen über ein Kohlenstoffatom gebundenen Piperidinylrest darstellt, übergeführt wird, oder

eine so erhaltene Verbindung der allgemeinen Formel I, in der $R_1$ einen über ein Kohlenstoffatom gebundenen durch ein oder zwei Halogenatome substituierten Pyridinrest darstellt, durch Halogenaustausch in eine entsprechende Verbindung der allgemeinen Formel I, in der $R_1$ einen über ein Kohlenstoffatom gebundenen Pyridinrest darstellt, der durch eine Amino- oder Morpholinogruppe und gegebenenfalls durch ein Halogenatom substituiert ist, übergeführt wird oder

eine so erhaltene Verbindung der allgemeinen Formel I, welche ein optisch aktives Kohlenstoffatom in Position 4 oder 5 des Pyridazinon-Ringes enthält, wenn $R_2$ eine Alkylgruppe darstellt, in ihre optisch aktiven Antipoden mittels Racematspaltung aufgetrennt wird oder

eine so erhaltene Verbindung der allgemeinen Formel I in ihre Säureadditionssalze, insbesondere in ihre physiologisch verträglichen Salze mit anorganischen oder organischen Säuren, übergeführt wird.

## Claims

1. Pyridazinones of general formula I

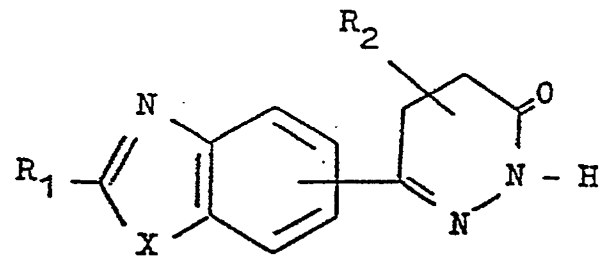

(I)

wherein

X represents an oxygen or sulphur atom,

$R_1$ represents a saturated 5- to 7-membered alkyleneimino ring bonded via a nitrogen atom or a 5- to 7-membered saturated imino, N-alkylimino, N-phenylalkylimino, N-alkanoylimino or N-alkoxycarbonyliminoalkylene ring bonded via a carbon atom, whilst the above-mentioned rings may be substituted by one or two alkyl groups in the carbon structure and a methylene group in the 4-position of a 6- or 7-membered ring may be replaced by an oxygen or sulphur atom or by a sulphinyl, sulphonyl, imino, alkylimino, phenylalkylimino, alkoxycarbonylimino or alkanoylimino group, or $R_1$ represents a saturated 5- to 7-membered alkyleneimino ring (optionally substituted by one or two alkyl groups) bonded via a nitrogen atom and in which a $-CH_2CH_2-$ group is replaced by an –NH–CO group, the –CO– group in this –NH–CO– group being linked to the N-atom already present, or $R_1$ represents a 5-membered heteroaromatic ring bonded via a nitrogen atom, which may additionally contain an oxygen or sulphur atom, one or two nitrogen atoms, or an oxygen or sulphur atom and a nitrogen atom and in which the heteroaromatic ring may be substituted by one or two alkyl groups, or in the case of a pyrazole, may alternatively be substituted by a hydroxy and an alkyl group, or $R_1$ represents a 5- or 6-membered heteroaromatic ring bonded via a carbon atom, in which the heteroaromatic ring may contain an oxygen or sulphur atom, one, two or three nitrogen atoms, or an oxygen or sulphur atom and one or two nitrogen atoms, whilst the heteroaromatic rings may be substituted by one or two alkyl groups, by an amino or alkanoylamino group or, in the case of a pyridine, by one or two halogen atoms or by a halogen atom and an amino or morpholino group, and

$R_2$ represents a hydrogen atom or an alkyl group; or

X represents an imino group optionally substituted by an alkyl or phenylalkyl group,

$R_1$ represents a saturated 5- to 7-membered alkyleneimino ring bonded via a nitrogen atom or a 5- to 7-membered saturated imino, N-alkylimino, N-phenylalkylimino, Nalkanoylimino or N-alkoxycarbonyliminoalkylene ring bonded via a carbon atom, whilst the above-mentioned rings may be substituted by one or two alkyl groups in the carbon structure and a methylene group in the 4-position of a 6- or 7-membered ring may be replaced by an oxygen or sulphur atom or by a sulphinyl, sulphonyl, imino, alkylimino, phenylalkylimino, alkoxycarbonylimino or alkanoylimino group, or $R_1$ represents a saturated 5- to 7-membered alkyleneimino ring (optionally substituied by one or two alkyl groups) bonded via a nitrogen atom and in which a $-CH_2CH_2-$ group is replaced by an –NH–CO group, th –CO– group in this –NH–CO– group being linked to the N-atom already present, and

$R_2$ represents a hydrogen atom or an alkyl group, whilst all the above-mentioned alkyl, alkanoyl or alkoxy groups may contain from 1 to 3 carbon atoms; or

X represents an imino group,

$R_1$ represents a 1-imidazolyl, 1-pyrazolyl, 3-methyl-1-pyrazolyl, 3-hydroxy-4-methyl-2-pyrazolyl or 1-[1.2.4]triazolyl group and

$R_2$ represents a methyl group in the 5-position; or

X represents an imino group,

$R_1$ represents a 2-chloro-6-morpholino-4-pyridyl group and

$R_2$ represents a hydrogen atom]

the optically active antipodes thereof, if $R_2$ does not represent hydrogen, and the acid addition salts thereof.

2. Pyridazinones of general formula I as claimed in claim 1

(wherein

X represents an oxygen or sulphur atom,

$R_1$ represents a pyrrolidinyl, piperidino, 1-hexahydroazepinyl, morpholino, thiomorpholino, 1oxidothiomorpholino, 1,1-dioxidothiomorpholino, 1-piperazinyl, 4-methyl-1-piperazinyl, 4-acetyl-1-piperazinyl, 4-carbethoxy-1-piperazinyl, 1-imidazolyl, 1-pyrazolyl, 1-[1.2.4]triazolyl, imidazolidin-2-on-1-yl or 3-hydroxy-2-pyrazolyl group bonded via a nitrogen atom, or a piperidino, N-acetylpiperidino, furyl, thienyl, pyrrolyl, pyridyl, aminopyridyl, acetylaminopyridyl, pyrimidyl, pyrazinyl, oxazolyl, dihalopyridyl or halomorpholinopyridyl group bonded via a carbon atom, whilst all the above mentioned groups may be substituted in the carbon structure by a methyl group, and

$R_2$ represents a hydrogen atom or a methyl group in the 5-position); or

X represents an imino group,

$R_1$ represents a pyrrolidinyl, piperidino, 1-hexahydroazepinyl, morpholino, thiomorpholino, 1-oxidothiomorpholino, 1,1-dioxidothiomorpholino, 1-piperazinyl, 4-methyl-1-piperazinyl, 4-acetyl-1-piperazinyl, 4-

carbethoxy-1-piperazinyl or imidazolidin-2-on-1-yl group bonded via a nitrogen atom, or a piperidino or N-acetyl-piperidino group bonded via a carbon atom, whilst all the above mentioned groups may be substituted in the carbon structure by a methyl group, and

$R_2$ represents a hydrogen atom or a methyl group in the 5-position; or

X represents an imino group,

$R_1$ represents a 1-imidazolyl, 1-pyrazolyl, 3-methyl-1-pyrazolyl, 3-hydroxy-4-methyl-2-pyrazolyl or 1-[1.2.4]triazolyl group and

$R_2$ represents a methyl group in the 5-position; or

X represents an imino group,

$R_1$ represents a 2-chloro-6-morpholino-4-pyridyl group and

$R_2$ represents a hydrogen atom)

the optically active antipodes thereof, if $R_2$ does not represent hydrogen, and the acid addition salts thereof.

3. Pyridazinones of general formula I as claimed in claim 1, wherein

X represents an oxygen atom,

$R_1$ represents a 1-pyrrolidinyl, 1-imidazolyl, 2-furyl, 3-thienyl, 1-piperazinyl, 4-methyl-1-piperazinyl, 4-carbethoxy-1-piperazinyl, 4-morpholino, 4-thiomorpholino, 1-oxido-4-thiomorpholino or 4-pyridyl group and

$R_2$ in the 5-position represents a methyl group: or

X represents an imino group,

$R_1$ represents a 1-pyrrolidinyl, 1-piperazinyl, 4-methyl-1-piperazinyl, 4-carbethoxy-1-piperazinyl, 4-morpholino, 4-thiomorpholino or 1-oxido-4-thiomorpholino group and

$R_2$ in the 5-position represents a methyl group, the optically active antipodes thereof, if $R_2$ does not represent hydrogen, and the acid addition salts thereof.

4. 5-Methyl-6-[2-(1-imidazolyl)-benzoxazol-5-yl]-4,5-dihydro-3(2H)-pyridazinone and its optically active enantiomers and acid addition salts thereof.

5. 5-Methyl-6-[2-(1-piperazinyl)-benzoxazol-6-yl]-4,5-dihydro-3(2H)-pyridazinone and its optically active enantiomers and acid addition salts thereof.

6. Physiologically acceptable acid addition salts of the compounds as claimed in any one of claims 1 to 5 with inorganic or organic acids.

7. Pharmaceutical compositions containing a compound of general formula I as claimed in claims 1 to 5 or a physiologically acceptable acid addition salt thereof as claimed in claim 6 in addition to one or more inert carriers and/or diluents.

8. Process for preparing a pharmaceutical composition as claimed in claim 7, characterised in that a compound as claimed in claims 1 to 5 or a physiologically acceptable acid addition salt thereof as claimed in claim 6 is incorporated in one or more inert carriers and/or diluents by a non-chemical method.

9. Use of a compound as claimed in claims 1 to 6 for the preparation of a pharmaceutical composition which is suitable for treating chronic heart insufficiency, Angina pectoris and for the prevention of arterial thromboembolism and arterial blockage disorders.

10. Process for preparing the compounds as claimed in claims 1 to 6, characterised in that

a) in order to prepare compounds of general formula I wherein $R_1$ represents one of the above-mentioned groups bonded by a carbon atom, a compound of general formula

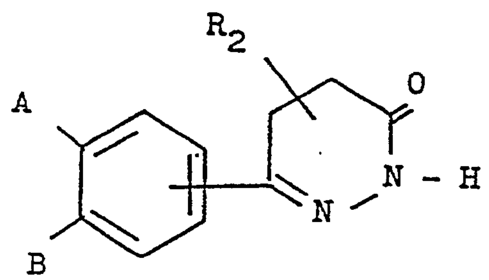

(II)

optionally formed in the reaction mixture, wherein $R_2$ is defined as in claims 1 to 3,
one of the groups A or B represents a

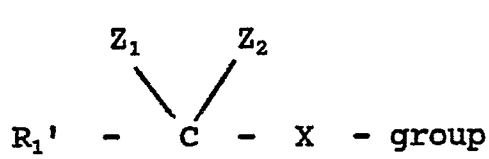

and the other group A or B represents an amino group or a

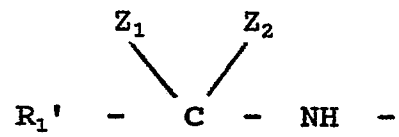

or

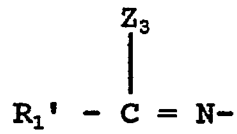

group

wherein X is defined as in claims 1 to 3,
$R_1'$ represents one of the groups bonded via a carbon atom mentioned for $R_1$ in claims 1 to 3, and
$Z_1$ and $Z_2$, which may be identical or different, represent nucleophilic leaving groups or
$Z_1$ and $Z_2$ together represent an oxygen or sulphur atom, an imino group optionally substituted by an alkyl or phenylalkyl group, whilst the above mentioned alkyl moieties may each contain 1 to 3 carbon atoms, or an alkylenedioxy group with 2 or 3 carbon atoms and
$Z_3$ has the meanings given for $Z_1$ hereinbefore or represents an imidazolyl group or a chlorine, bromine or iodine atom,
is cyclised) or
b) a carboxylic acid of general formula

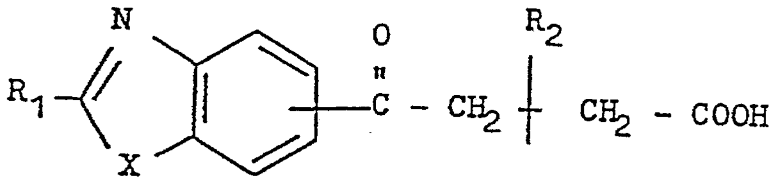

(III)

(wherein $R_1$ and $R_2$ are defined as in claims 1 to 3) or the reactive acid derivatives thereof are reacted with hydrazine or
c) in order to prepare compounds of general formula I wherein $R_1$ represents one of the groups bonded by a nitrogen atom mentioned in claims 1 to 3, a compound of general formula

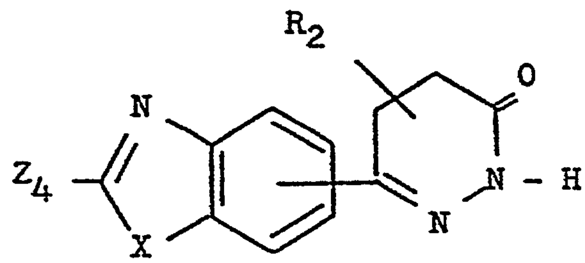

(IV)

(wherein $R_2$ and X are defined as in claims 1 to 3 and
$Z_4$ represents a nucleophilically exchangeable group) is reacted with an amine of general formula

$$H\text{–}R_1'' \qquad (V)$$

wherein $R_1''$ represents one of the groups bonded by a nitrogen atom mentioned for $R_1$ in claims 1 to 3, or
d) in order to prepare compounds of general formula I wherein X represents an oxygen atom or an imino group optionally substituted by an alkyl or phenylalkyl group, a compound of general formula

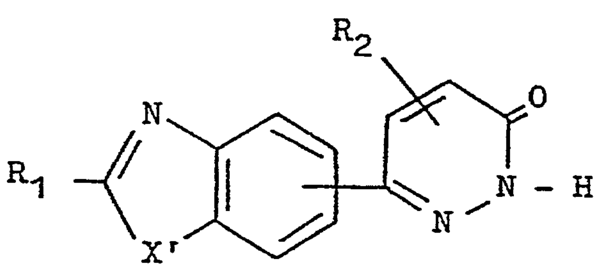

(VI)

(wherein $R_1$ and $R_2$ are defined as in claims 1 to 3 and X, represents an oxygen atom or an imino group optionally substituted by an alkyl or phenylalkyl group, whilst the alkyl group may contain from 1 to 5 carbon atoms) is hydrogenated and
subsequently if desired a compound of general formula I thus obtained wherein $R_1$ contains an imino and/or amino group, is converted by alkanoylation into a corresponding compound of general formula I

wherein $R_1$ contains an alkanoylimino and/or alkanoylamino group, or
a compound of general formula I thus obtained wherein $R_1$ represents a carbon bound, halogen-substituted pyridine group is converted by catalytic dehalogenation and hydrogenation into a compound of general formula I wherein $R_1$ represents a piperidinyl group bonded via a carbon atom, or
a compound of general formula I thus obtained wherein $R_1$ represents a pyridine group substituted by 1 or 2 halogen atoms and bonded via a carbon atom, is converted by halogen exchange into a corresponding compound of general formula I wherein $R_1$ represents a pyridine group bonded via a carbon atom and substituted by an amino or morpholino group and optionally by a halogen atom, or
a compound of general formula I thus obtained which contains an optically active carbon atom in position 4 or 5 of the pyridazinone ring, if $R_2$ represents an alkyl group, is converted into the optically active antipodes thereof by racemate splitting or
a compound of general formula I thus obtained is converted into the acid addition salts thereof, more particularly the physiologically acceptable salts thereof with inorganic or organic acids.

**Revendications**

1. Pyridazinones de formule générale

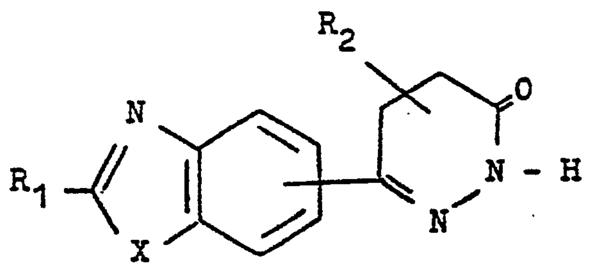

,(I)

dans laquelle
X représente un atome d'oxygène ou de soufre,
$R_1$ représente un cycle alcoylèneimino, satguré, à 5 à 7 membres, lié par l'intermédiaire d'un atome d'zote ou représente un cycle imino-, N-alcoxyl-imino-, N-phénylalcoyl-imino-, N-alcanoyl-imino- ou N-alcoxycarbonyl-imino-alcoylène saturé, à 5 à 7 membres, lié par l'intermédiaire d'un atome de carbone, les cycles mentionnés ci-dessus pouvant être substitués dans le squelette carboné par un ou deux groupes alcoyle et un groupe méthylène en position 4 d'un cycle à 6 ou 7 membres pouvant être remplacé par un atome d'oxygène ou de soufre, un groupe sulfinyle, sulfonyle, imino, alcoylimino, phénylalcoylimino, alcoxycarbonylimino ou alcanoylimino, ou représente un cycle alcoylènimino, saturé à 5 à 7 membres, éventuellement substitué par un ou deux groupes alcoyle, lié par l'intermédiaire d'un atome d'azote, cycle dans lequedl un groupe $-CH_2CH_2-$ est remplacé par un groupe –NH–CO–, le groupe CO de ce groupe –NH–CO– étant relié à l'atome de N déjà présent, ou représente un cycle hétéreo-atomatique à 5 membres, lié par l'intermédiaire d'un atome d'azote, lequel cycle peut contenir en outre un atome d'oxygèn ou de soufre, un ou deux atomes d'azote, un atome d'oxygène ou de soufre et un atome d'azote, le cycle hétéroaromatique pouvant être substitué par un ou deux groupes alcoyle, et dans le cas des pyrazoles également par un groupe hydroxy et un groupe alcoyle, ou représente un cycle hétéro-aromatique à 5 ou 6 membres, lié par l'intermédiaire d'un atome de carbone, lequel cycle peut contenir un atome d'oxygène ou de soufre, un, deux ou trois atomes d'azote, un atome d'oxygène ou de soufre et un ou deux atomes d'azote, les cycles hétéro-aromatiques pouvant être substitués par un ou deux groupes alcoyle, par un groupe amino ou alcanoylamino et, dans le cas des pyridines, également par un ou deux atomes d'halogène ou par un atome d'halogène et un groupe amino ou morpholino, et
$R_2$ représente un atome d'hydrogène ou un groupe alcoyle ou
X représente un groupe imino éventuellement substitué par un groupe alcoyle ou phénylalcoyle,
$R_1$ représente un cycle alcoylènimino à 5 à 7 membres, saturé, lié par l'intermédiaire d'un atome d'azote ou représente un groupe imino-, N-alcoylimino-, N-phénylalcoylimino-, N-alcanoylimino- ou N-alcoxycarbonylimino-alcoylène, saturé, à 5 à 7 membres, lié par l'intermédiaire d'un atome de carbone, les cycles mentionnés ci-dessus pouvant être substitués dans le squelette carboné par un ou deux groupes alcoyle et un groupe méthylène en position 4 d'un cycle à 6 ou 7 membres pouvant être remplacé par un atome d'oxygène ou de soufre, un groupe sulfinyle, sulfonyle, imino, alcoyolimino, phénylalcoylimino, alcoxycarbonylimino ou alcanoylimino, ou représente un cycle alcoylènimino à 5 à 7 membres, saturé, éventuellement substitué par un ou deux groupes alcoyle, lié par l'intermédiaire d'un atome d'azote, cycle dans lequel un groupe $-CH_2CH_2-$ est remplacé par un groupe –NH–CO–, le groupe CO de ce groupe –NH–CO– étant relié à l'atome de N déjà présent, et
$R_2$ représente un atome d'hydrogène ou un groupe alcoyle, tous les groupes alcoyle, alcanoyle ou alcoxy mentionnés ci-dessus pouvant contenir chacun 1 à 3 atomes de carbone, ou
X représente un groupe imino,

$R_1$ représente un groupe 1-imidazolyle, 1-pyrazolyle, 3-méthyl-1-pyrazolyle, 3-hydroxy-4-méthyl-2-pyrazolyle ou 1-[1.2.4]triazolyle et
$R_2$ représente un groupe méthyle en position 5 ou
X représente un groupe imino,
$R_1$ représente un groupe 2-chloro-6-morpholino-4-pyridyle et
$R_2$ représente un atome d'hydrogène,
leurs antipodes optiquement actifs, lorsque $R_2$ ne représente pas un atome d'hydrogène, et leurs sels d'addition d'acides.

2. Pyridazinones de formule générale I selon la revendication 1, dans laquelle
X représente un atome d'oxygène ou de soufre,
$R_1$ représente un groupe pyrrolidinyle, pipéridino, 1-hexahydroazépinyle, morpholino, thiomorpholino, 1-oxydothiomorpholino, 1,1-dioxydothiomorpholino, 1-pipérazinyle, 4-méthyl-1-pipérazinyle, 4-acétyl-1-pipérazinyle, 4-carbéthoxy-1-pipérazinyle, 1-imidazolyle, 1-pyrazolyle, 1-[1.2.4]triazolyle, imidazolidine-2-one-1-yle ou 3-hydroxy-2-pyrazolyle lié par l'intermédiaire d'un atome d'azote ou représente un groupe pipéridino, N-acétylpipéridino, furyle, thiényle, pyrrolyle, pyridyle, amino-pyridyle, acétylaminopyridyle, pyrimidyle, pyrazinyle, oxazolyle, dihalogéno-pyridyle ou halogéno-morpholino-pyridyle, lié par l'intermédiaire d'un atome de carbone, tous les radicaux mentionnés ci-dessus pouvant être substitués dans le squelette carboné par un groupe méthyle, et
$R_2$ représente un atome d'hydrogène ou un groupe méthyle en position 5 ou
X représente le groupe imino,
$R_1$ représente un groupe pyrrolidinyle, pipéridino, 1-hexahydroazépinyle, morpholino, thiomorpholino, 1-oxydothiomorpholino, 1,1-dioxydothiomorpholino, 1-pipérazinyle, 4-méthyl-1-pipérazinyle, 4-acétyl-1-pipérazinyle, 4-carbéthoxy-1-pipérazinyle ou imidazolidine-2-one-1-yle, lié par l'intermédiaire d'un atome d'azote ou représente un groupe pipéiridino ou N-acétyl-pipéridino, lié par l'intermédiaire d'un atome de carbone, tous les radicaux mentionnés ci-dessus pouvant être substitués dans le squelette carboné par un groupe méthyle, et
$R_2$ représente un atome d'hydrogène ou un groupe méthyle en position 5 ou
X représente un grope imino,
$R_1$ représente un groupe 1-imidazolyle, 1-pyrazolyle, 3-méthyl-1-pyrazolyle, 3-hydroxy-4-méthyl-2-pyrazolyle ou 1-[1.2.4]triazolyle et
$R_2$ représente un groupe méthyle en position 5 ou
X représente un groupe imino,
$R_1$ représente un groupe 2-chloro-6-morpholino-4-pyridyle et
$R_2$ représente un atome d'hydrogène
leurs antipodes optiquement actifs, lorsque R2 ne représente pas un atome d'hydrogène, et leurs sels d'addition d'acides.

3. Pyridazinones de formule générale I selon la revendication 1, dans laquelle
X représente un atome d'oxygène,
$R_1$ représente le groupe 1-pyrrolidinyle, 1-imidazolyle, 2-furyle, 3-thiényle, 1-pipérazinyle, 4-méthyl-1-pipérazinyle, 4-carbéthoxy-1-pipérazinyle, 4-morpholino, 4-thiomorpholino, 1-oxydo-4-thiomorpholino ou 4-pyridyle et
$R_2$ en position 5 représente le groupe méthyle ou
X représente le groupe imino,
$R_1$ représente le groupe 1-pyrrolidinyle, 1-pipérazinyle, 4-méthyl-1-pipérazinyle, 4-carbéthoxy-1-pipérazinyle, 4-morpholino, 4-thiomorpholino ou 1-oxydo-4-thiomorpholino et
$R_2$ en position 5 représente le groupe méthyle, leurs antipodes optiquement actifs, lorsque $R_2$ ne représente pas un atome d'hydrogène, et leurs sels d'addition d'acides.

4. La 5-méthyl-6-[2-(1-imidazolyl)-benzoxazol-5-yl]-4,5-dihydro-3(2H)-pyridazinone, ses antipodes optiquement actifs et ses sels d'addition d'acides.

5. La 5-méthyl-6-[2-(1-pipérazinyl)-benzoxazol-6-yl]-4,5-dihydro-3(2H)-pyridazinone, ses antipodes optiquement actifs et ses sels d'addition d'acides.

6. Sels d'addition d'acides physiologiquement supportables des composés selon les revendications 1 à 5 avec des acides minéraux ou organiques.

7. Médicament contednant un composé de formule générale I selon les revendications 1 à 5 ou son sels d'addition d'acide physiologiquement supportable selon la revendication 6, conjointement à un ou plusieurs excipients et/ou diluants inertes.

8. Procédé pour la fabrication d'un médicament selon la revendication 7, caractérisé en ce qu'on introduit, par voie non chimique, un composé selon les revendications 1 à 5 ou son sel d'addition d'acide physiologiquement supportable selon la revendication 6, dans un ou plusieurs excipients et/ou diluants inertes.

9. Utilisation d'un composé selon les revendications 1 à 6 pour la fabrication d'un médicament, lequel convient au tritement de l'insuffisance cardiaque chronique, de l'angine de poitrine et à la prophylaxie des thromboembolies artérielles et des maladies occlusives des artères.

10. Procédé pour la préparation des composés selon les revendications 1 à 6, caractérisé en ce que
a) pour la préparation de composés de formule générale I dans laquelle $R_1$ représente l'un des radicaux liés par l'intermédiaire d'un atome de carbone, mentionnés au début, on cyclise un composé éventuellement formé dans le mélange réactionnel, de formule générale

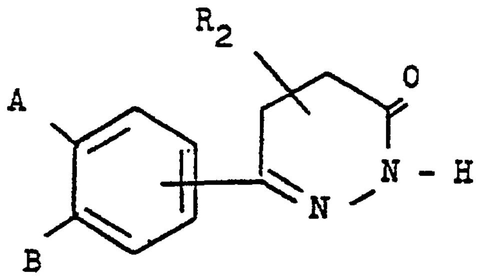

,(II)

dans laquelle
$R_2$ est défini comme dans les revendications 1 à 3, l'un des radicaux A ou B représente un groupe

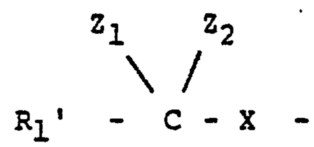

et

l'autre des radicaux A ou B représente un groupe amino ou un groupe

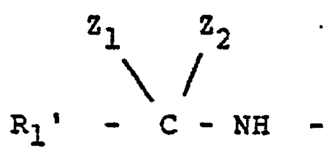

ou

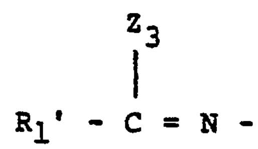

X étant défini comme dans les revendications 1 à 3 et
$R_1'$ représente l'un des radicaux liés par l'intermédiaire d'un atome de carbone, mentionnés dans les revendications 1 à 3 pour $R_1$
$Z_1$ et $Z_2$, qui peuvent être identiques ou différents, représentent des groupes partants nucléophiles
ou
$Z_1$ et $Z_2$ représentent ensemble un atome d'oxygène ou de soufre, un groupe imino éventuellement substitué par un groupe alcoyle ou phénylalcoyle, les parties alcoyle mentionnées ci-dessus pouvant contenir dans chaque cas 1 à 3 atomes de carbone, ou représentent un groupe alcoylènedioxy avec 2 ou 3 atomes de carbone et
$Z_3$ possède les significations mentionnées ci-dessus pour $Z_1$ ou représente un groupe imidazolyle, un atome de chlore, de brome ou d'iode, ou
b) on fait réagir un acide carboxylique de formule générale

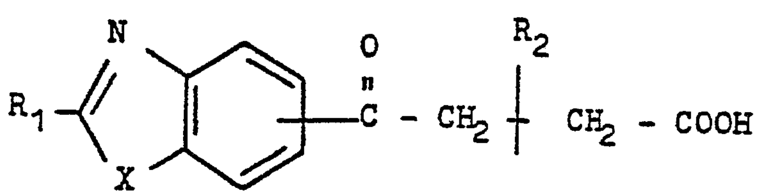

,(III)

dans laquelle
$R_1$ et $R_2$ sont définis comme dans les revendications 1 à 3, ou leurs dérivés acides réactifs, avec l'hydrazine ou
c) pour la préparation de composés de formule générale I dans laquelle $R_1$ représente l'un des radicaux liés par l'intermédiaire d'un atome d'azote, mentionnés dans les revendications 1 à 3, on fait réagir un composé de formule générale

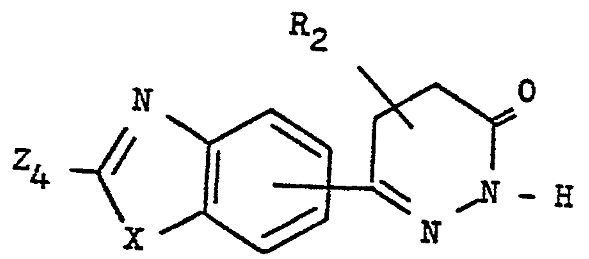

, (IV)

dans laquelle
$R_2$ et X sont définis comme dans les revendications 1 à 3 et
$Z_4$ représente un groupe échangeable de façon nucléophile, avec un amine de formule générale

$$H{-}R_1'', \qquad (V)$$

dans laquelle
R1" représente l'un des radicaux liés par l'intermédiaire d'un atome d'azote, mentionnés dans les revendications 1 à 3 pour $R_1$, ou
d) pour la préparation de composés de formule générale I dans laquelle X représente un atome d'oxygène ou un groupe imino, éventuellement substitué par un groupe alcoyle ou phénylalcoyle, on hydrogène un composé de formule générale

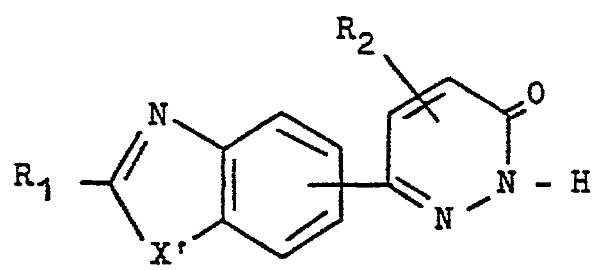

, (VI)

dans laquelle
$R_1$ et $R_2$ sont définis comme dans les revendications 1 à 3, et
X' représente un atome d'oxygène ou un groupe imino, éventuellement substitué par un groupe alcoyle ou phénylalcoyle, la partie alcoyle pouvant dans chaque cas contenir 1 à 5 atomes de carbone et
si on le désire, on transforme ensuite un composé ainsi obtenu de formule générale I, dans laquelle $R_1$ contient un groupe imino et/ou amino, au moyen d'une alcanoylation, en un composé correspondant de formule générale I, dans laquelle $R_1$ contient un groupe alcanoylimino et/ou alcanoylamino, ou
on transforme un composé ainsi obtenu de formule générale I, dans laquelle $R_1$ représente un radical pyridine, lié par l'intermédiaire d'un atome de carbone, substitué par des atomes d'halogène, au moyen d'une déshalogénation catalytique et d'une hydrogénation en un composé de formule générale I dans laquelle $R_1$ représente un radical pipéridinyle lié par l'intermédiaire d'un atome de carbone, ou
on transforme un composé ainsi obtenu de formule générale I dans laquelle $R_1$ représente un radical pyridine, lié par l'intermédiaire d'un atome de carbone, substitué par un ou deux atomes d'halogène, par échange d'halogènes en un composé correspondant de formule générale I dans laquelle $R_1$ représente un radical pyridine lié par l'intermédiaire d'un atome de carbone, lequel radical est substitué par un groupe amino ou morpholino et éventuellement par un atome d'halogène, ou
on sépare un composé ainsi obtenu de formule générale I, qui contient, en position 4 ou 5 du cycle pyridazinone, un atome de carbone optiquement actif, lorsque $R_2$ représente un groupe alcoyle, en ses antipodes optiquement actifs, au moyen d'un clivage de racémate ou
on transforme un composé ainsi obtenu de formule générale I en ses sels d'addition d'acides, en particulier en ses sels physiologiquement supportables avec des acides minéraux ou organiques.